(19) Europäisches Patentamt
European Patent Office
Office européen des brevets

(11) Numéro de publication : **0 076 199**
**B1**

(12) # FASCICULE DE BREVET EUROPÉEN

(45) Date de publication du fascicule du brevet : **30.12.86**

(21) Numéro de dépôt : **82401697.6**

(22) Date de dépôt : **20.09.82**

(51) Int. Cl.⁴ : **C 07 D487/04**, A 61 K 31/495, A 61 K 31/535, A 61 K 31/55 // (C07D487/04, 249:00, 239:00)

(54) **Nouvelles triazoloquinazolones et leurs sels, procédé et intermédiaires de préparation, leur application comme médicaments et les compositions les renfermant.**

(30) Priorité : **24.09.81 GB 8128875**

(43) Date de publication de la demande : **06.04.83 Bulletin 83/14**

(45) Mention de la délivrance du brevet : **30.12.86 Bulletin 86/52**

(84) Etats contractants désignés : **AT BE CH DE FR IT LI LU NL SE**

(56) Documents cités :
**EP-A- 0 034 529**
**DD-A- 139 715**
**Le dossier contient des informations techniques présentées postérieurement au dépôt de la demande et ne figurant pas dans le présent fascicule.**

(73) Titulaire : **ROUSSEL-UCLAF**
**35, boulevard des Invalides**
**F-75007 Paris (FR)**

(72) Inventeur : **Tully, Wilfred Roger**
**5, St Peter's Road**
**Cirencester, Glouc. (GB)**
Inventeur : **Westwood, Robert**
**8, Fernham Road**
**Faringdon, Oxon (GB)**
Inventeur : **Rowlands, David Alun**
**Wyke House Crudwell**
**Malmesbury Wiltshire (GB)**
Inventeur : **Clements-Jewery, Stephen**
**56, High Street**
**Blunsdon Nr. Swindon Wiltshire (GB)**

(74) Mandataire : **Vieillefosse, Jean-Claude et al**
**Département des Brevets ROUSSEL UCLAF B.P no 9**
**F-93230 Romainville (FR)**

**EP 0 076 199 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouvelles triazoloquinazolones et leurs sels, ainsi que le procédé de préparation, l'application à titre de médicaments de ces nouveaux produits, les compositions les renfermant et les nouveaux intermédiaires obtenus.

L'invention a pour objet de nouvelles triazoloquinazolones, ainsi que leurs sels, caractérisées en ce qu'elles répondent à la formule générale (I) :

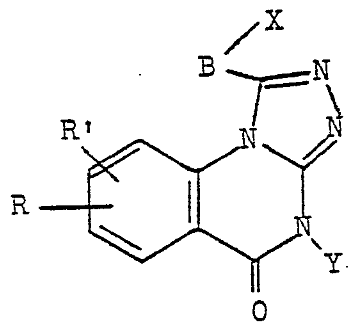

(I)

dans laquelle,

R et R', identiques ou différents, représentent un atome d'hydrogène, un atome d'halogène, un radical alcoyle renfermant de 1 à 3 atomes de carbone, un radical alcoxy renfermant de 1 à 3 atomes de carbone, un radical nitro,

Y représente un radical alcoyle, linéaire ou ramifié, renfermant de 1 à 6 atomes de carbone, un radical cycloalcoyle renfermant de 3 à 8 atomes de carbone, un radical alcényle renfermant de 2 à 4 atomes de carbone, un radical aryle renfermant de 6 à 8 atomes de carbone ou aralcoyle renfermant 7 ou 8 atomes de carbone,

B représente un groupement

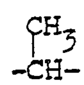

ou un radical alcoylène $—(CH_2)n$- dans lequel n représente un nombre entier égal à 1, 2 ou 3,

X représente un groupement

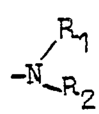

dans lequel $R_1$ et $R_2$, identiques ou différents, représentent un atome d'hydrogène, un groupement alcoyle, linéaire ou ramifié, renfermant de 1 à 6 atomes de carbone, un radical cycloalcoyle renfermant de 3 à 8 atomes de carbone, un radical aryle ou aralcoyle renfermant de 6 à 8 atomes de carbone, un radical aminoalcoyle, monoalcoyl ou dialcoylaminoalcoyle dans lesquels les groupements alcoyles renfermant de 2 à 4 atomes de carbone, un radical pipéridinoalcoyle, morpholino-alcoyle ou pipérazinylalcoyle, où $R_1$ et $R_2$ forment ensemble avec l'atome d'azote auxquels ils sont liés, un hétérocycle saturé mono ou bicyclique renfermant de 4 à 8 atomes de carbone pouvant être substitué par 1 ou 2 radicaux méthyle, ou pouvant renfermer en outre un atome d'oxygène, de soufre ou d'azote, ledit atome d'azote pouvant être substitué par un radical alcoyle renfermant de 1 à 3 atomes de carbone, par un radical hydroxyalcoyle renfermant de 1 à 3 atomes de carbone, par un radical aryle ou aralcoyle renfermant de 6 à 8 atomes de carbone ou par un radical alcoxy carbonyle renfermant de 2 à 5 atomes de carbone, ou enfin, X représente un radical

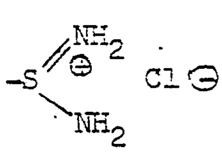

ou

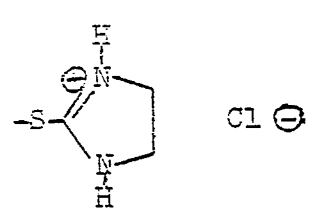

Dans la formule générale (I) et dans ce qui suit,

— le terme atome d'halogène désigne par exemple un atome de fluor, de chlore ou de brome et de préférence un atome de chlore ;

— le terme radical alcoyle renfermant de 1 à 3 atomes de carbone désigne un radical méthyle, éthyle, propyle ou isopropyle ;

— le terme radical alcoxy renfermant de 1 à 3 atomes de carbone désigne un radical méthoxy, éthoxy, propoxy ou isopropoxy ;

— le terme radical alcoyle linéaire ou ramifié renfermant de 1 à 6 atomes de carbone désigne par exemple un radical méthyle, éthyle, propyle, isopropyle, butyle, iso-butyle, tert-butyle, pentyle, hexyle ;

— le terme radical cycloalcoyle renfermant de 3 à 8 atomes de carbone désigne par exemple un radical cyclopropyle, cyclopentyle ou cyclohexyle ;

— le terme radical alcényle renfermant de 2 à 4 atomes de carbone désigne par exemple un radical vinyle ou allyle :

— le terme radical aryle renfermant de 6 à 8 atomes de carbone ou aralcoyle renfermant 7 ou 8 atomes de carbone désigne par exemple un radical phényle, benzyle ou phénéthyle ;

— le terme radical aminoalcoyle désigne par exemple un radical aminoéthyle ;

— le terme radical monoalcoyl ou dialcoyl-aminoalcoyle dans lesquels les groupements alcoyles renferment de 2 à 4 atomes de carbone, désigne, par exemple, un radical méthylaminoéthyle, diméthylaminoéthyle ;

— le terme radical pipéridinoalcoyle, morpholinoalcoyle ou pipérazinylalcoyle désigne par exemple un radical pipéridinoéthyle, morpholinoéthyle ou pipérazinyléthyle ;

— le terme hétérocycle saturé mono ou bicyclique renfermant de 4 à 8 atomes de carbone pouvant être substitué par 1 ou 2 radicaux méthyle et pouvant renfermer en outre un atome d'oxygène, de soufre ou d'azote désigne, par exemple, un radical pyrrolidinyle, pipéridino, 2,3,4,5,6,7-hexa-hydroazépino, 3-aza bicyclo [3,2,2] nonano, 2,6-diméthylpipéridino, 3,5-diméthylpipéridino, ou un radical morpholino, thiomorpholino ou pipérazin-1-yl ;

— le terme radical hydroxyalcoyle renfermant de 1 à 3 atomes de carbone désigne par exemple un radical hydroxyméthyle, hydroxyéthyle ou hydroxypropyle ;

— le terme radical alcoxycarbonyle renfermant de 2 à 5 atomes de carbone désigne, par exemple, un radical méthoxy ou éthoxy carbonyle.

Les sels d'addition avec les acides minéraux ou organiques peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, alcanesulfonique, tels que l'acide méthane sulfonique et arylsulfoniques, tels que l'acide benzène sulfonique.

Parmi les produits, objet de l'invention, on peut citer notamment, les dérivés répondant à la formule (I) ci-dessus, ainsi que leurs sels, caractérisés en ce que dans ladite formule (I), R et R', identiques ou différents, représentent un atome d'hydrogène, un atome de chlore, un radical méthyle, un radical méthoxy ou un radical nitro,

Y représente un radical alcoyle, linéaire ou ramifié, renfermant de 1 à 6 atomes de carbone, un radical cyclohexyle, un radical allyle, un radical phényle ou benzyle,

B représente un radical méthylène ou éthylène,

X a la signification déjà indiquée.

Parmi les produits, objet de l'invention, on retient plus particulièrement les dérivés répondant à la formule (I) ci-dessus, ainsi que leurs sels, caractérisés en ce que dans ladite formule (I), R et R' représentent un atome d'hydrogène,

Y représente un radical méthyle, éthyle, n-propyle, iso-propyle, n-butyle, tert-butyle, n-hexyle, cyclohexyle, allyle, phényle ou benzyle,

B représente un radical méthylène ou éthylène,

X représente un groupement amino, diméthylamino, diéthylamino, dipropylamino, diisopropylamino, dibutylamino, diisobutylamino, butylamino, cyclohexylamino, pipéridinoéthylamino, pyrrolidinyle, pipéridino, 2,3,4,5,6,7-hexahydroazépino, 3-azabicyclo [3,2,2] nonano, 2,6-diméthylpipéridino, 3,5-diméthylpipéridino, morpholino, pipérazin-1-yl méthyl pipérazin-1-yl, hydroxyéthyl-pipérazin-1-yl, phénylpipérazin-1-yl, éthoxycarbonyle pipérazin-1-yl.

Parmi ces derniers, on peut citer, tout particulièrement, les dérivés répondant à la formule générale (I) ci-dessus, ainsi que leurs sels, caractérisés en ce que dans ladite formule (I), R et R' représentent un atome d'hydrogène, Y représente un radical méthyle, éthyle, n-propyle, iso-propyle, n-butyle, tert-butyle, n-hexyle, cyclohexyle, allyle, phényle ou benzyle, B représente un radical méthylène, X représente un groupement pyrrolidinyle, pipéridino, 2,3,4,5,6,7-hexahydrozépino, 3-azabicyclo [3,2,2] nonano, 2,6-diméthylpipéridino, 3,5-diméthylpipéridino et notamment :

La 1-pipéridinométhyl-4-éthyl [1,2,4] triazolo [4,3-a] quinazolin-5 (4H) one et ses sels ;

La 1-pipéridinométhyl-4-n-propyl [1,2,4] triazolo [4,3-a] quinazolin-5 (4H) one et ses sels ;

La 1-pipéridinométhyl-4-isopropyl [1,2,4] triazolo [4,3-a] quinazolin-5 (4H) one et ses sels ;

La 1-pipéridinométhyl-4-n-butyl [1,2,4] triazolo [4,3-a] quinazolin-5 (4H) one et ses sels ;

La 1-(2,3,4,5,6,7-hexahydroazépino) méthyl-4-n-butyl [1,2,4] triazolo [4,3-a] quinazolin-5 (4H) one et

ses sels ;

La 1-pipéridinométhyl 4-allyl [1,2,4] triazolo [4,3-a] quinazolin-5-(4H) one et ses sels.

L'invention a également pour objet un procédé de préparation des produits tels que définis par la formule (I) ci-dessus ainsi que de leurs sels, caractérisé en ce que l'on fait réagir un produit de formule (II) :

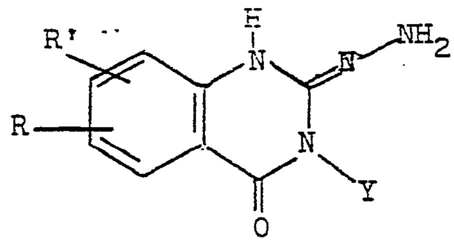

(II)

dans laquelle R, R' et Y ont la signification déjà indiquée, avec le dérivé halogéné d'un halogénure d'acyle de formule (III) :

$$Hal—B—CO—Hal_1 \quad (III)$$

dans laquelle Hal et $Hal_1$ représentent un atome de chlore ou de brome et B a la signification déjà indiquée, pour obtenir un produit de formule (IV) :

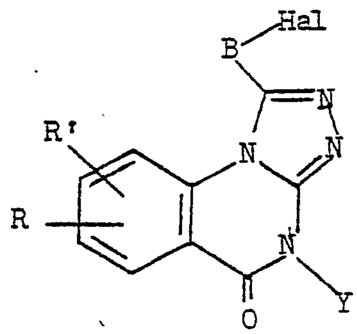

(IV)

dans laquelle R, R', Y, B et Hal ont la signification déjà indiquée, le cas échéant après avoir isolé puis cyclisé le produit intermédiaire de formule ($II_a$) :

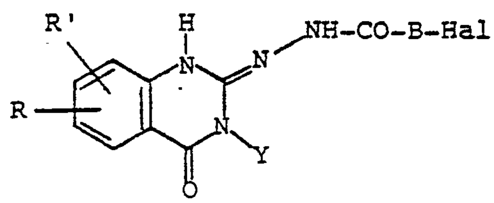

(IIa)

dans laquelle R, R', Y, B et Hal ont la signification déjà indiquée, puis fait réagir le produit de formule (IV) avec un produit de formule (V) :

$$H—X \quad (V)$$

dans laquelle X a la signification déjà indiquée, pour obtenir le produit de formule (I) recherché que l'on peut, le cas échéant, salifier.

Dans des conditions préférentielles de mise en œuvre de l'invention, le procédé de préparation ci-dessus décrit est caractérisé en ce que :

a) la réaction du produit de formule (II) avec le dérivé halogéné de l'halogénure d'acyle de formule (III) est effectuée au sein d'un solvant organique tel que le diméthylformamide et, le cas échéant, en présence d'un agent alcalin ;

b) le dérivé halogéné de l'halogénure d'acyle de formule (III) est le dérivé chloré du chlorure d'acyle :

c) la cyclisation du produit de formule ($II_a$) est effectuée par chauffage en présence d'un acide ;

d) la réaction du produit de formule (IV) avec le produit de formule (V) est effectuée au sein d'un solvant organique tel que le toluène et, le cas échéant, en présence d'un agent alcalin.

Selon une variante du procédé de préparation décrit ci-dessus, les produits de formule (I) dans

laquelle Y représente un radical alcoyle renfermant de 2 à 4 atomes de carbone, peuvent également être préparés par réduction d'un produit de formule (I) dans laquelle Y représente un radical alcényle renfermant de 2 à 4 atomes de carbone.

L'agent de réduction est, de préférence, l'hydrogène en présence d'un catalyseur, par exemple le palladium.

Les sels d'addition avec les acides des produits de formule (I), peuvent être préparés en faisant réagir lesdits produits de formule (I) avec un acide tel que ceux décrits précédemment, de préférence en quantité équimoléculaire.

L'invention a également pour objet, à titre de produits industriels nouveaux, utiles notamment pour préparer les produits de formule (I), les produits de formule (IV) dans laquelle R, R', Y, B et Hal ont la signification déjà indiquée.

Les produits, objet de la présente invention, possèdent de très intéressantes propriétés pharmacologiques ; ils sont doués notamment de remarquables propriétés antihistaminiques et bronchospasmolytiques.

Ces propriétés sont illustrées plus loin dans la partie expérimentale.

Ces propriétés justifient l'utilisation des nouvelles triazoloquinazolones, objet de l'invention, ainsi que de leurs sels pharmaceutiquement acceptables, à titre de médicaments.

La présente demande a ainsi également pour objet l'application, à titre de médicaments, des nouvelles triazoloquinazolones telles que définies par la formule (I) ainsi que de leurs sels pharmaceutiquement acceptables.

Parmi les médicaments, objet de l'invention, on retient de préférence, les médicaments, caractérisés en ce qu'ils sont constitués par les nouvelles triazoloquinazolones répondant à la formule (I) dans laquelle

R et R', identiques ou différents, représentent un atome d'hydrogène, un atome de chlore, un radical méthyle, un radical méthoxy ou un radical nitro,

Y représente un radical alcoyle, linéaire ou ramifié, renfermant de 1 à 6 atomes de carbone, un radical cyclohexyle, un radical allyle, un radical phényle ou benzyle,

B représente un radical méthylène ou éthylène,

X a la signification déjà indiquée ainsi que par leurs sels pharmaceutiquement acceptables.

Parmi les médicaments, objet de l'invention, on retient plus particulièrement ceux répondant à la formule (I), caractérisés en ce que dans ladite formule (I),

R et R' représentent un atome d'hydrogène,

Y représente un radical méthyle, éthyle, n-propyle, isopropyle, n-butyle, tert-butyle, n-hexyle, cyclohexyle, allyle, phényle ou benzyle,

B représente un radical méthylène ou éthylène,

X représente un groupement amino, diméthylamino, diéthylamino, dipropylamino, diisopropylamino, dibutylamino, diisobutylamino, butylamino, cyclohexylamino, pipéridinoéthylamino, pyrrolidinyle, pipéridino, 2,3,4,5,6,7-hexahydroazépino, 3-azabicyclo [3,2,2] nonano, 2,6-diméthylpipéridino, 3,5-diméthylpipéridino, morpholino, pipérazin-1-yl, méthylpipérazin-1-yl, hydroxyéthyl-pipérazin-1-yl, phénylpipérazin-1-yl, éthoxycarbonyle pipérazin-1-yl ainsi que leurs sels pharmaceutiquement acceptables.

Parmi ces derniers on peut citer tout particulièrement ceux répondant à la formule (I) dans laquelle R et R' représentent un atome d'hydrogène, Y représente un radical méthyle, éthyle, n-propyle, isopropyle, n-butyle, tert-butyle, n-hexyle cyclohexyle, allyle, phényle ou benzyle, B représente un radical méthylène, X représente un groupement pyrrolidinyle, pipéridino, 2,3,4,5,6,7-hexahydroazépino, 3-azabicyclo [3,2,2] nonano, 2,6-diméthylpipéridino, 3,5-diméthylpipéridino ainsi que leurs sels pharmaceutiquement acceptables et notamment les dérivés dont les noms suivent :

la 1-pipéridinométhyl-4-éthyl [1,2,4] triazolo [4,3-a] quinazolin-5 (4H) one et ses sels ;

la 1-pipéridinométhyl-4-n-propyl [1,2,4] triazolo [4,3-a] quinazolin-5 (4H) one et ses sels ;

la 1-pipéridinométhyl-4-isopropyl [1,2,4] triazolo [4,3-a] quinazolin-5 (4H) one et ses sels ;

la 1-pipéridinométhyl-4-n-butyl [1,2,4] triazolo [4,3-a] quinazolin-5(4H) one et ses sels ;

la 1-(2,3,4,5,6,7-hexahydroazepino) méthyl-4-n-butyl [1,2,4] triazolo [4,3-a] quinazolin-5 (4H) one et ses sels ;

la 1-pipéridinométhyl 4-allyl [1,2,4] triazolo [4,3-a] quinazolin-5 (4H) one et ses sels.

Ces médicaments trouvent, par exemple, leur emploi dans le traitement de l'asthme, de la bronchite et des désordres allergiques.

La dose usuelle, variable selon le produit utilisé, le sujet traité et l'affection en cause peut être, par exemple, de 2 mg à 2 g par jour, par voie orale.

L'invention a enfin pour objet les compositions pharmaceutiques qui renferment au moins un dérivé précité ou l'un de ces sels d'addition avec les acides pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive, parentérale ou locale.

Ces compositions pharmaceutiques peuvent être, par exemple, solides ou liquides et se présenter sous les formes phamaceutiques couramment utilisées en médecine humaine, comme par exemple, les

comprimés, simples ou dragéifiés, les gélules, les capsules, les granulés, les suppositoires, les préparations injectables, les aérosols ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs, les agents propellants.

Les produits de la formule (II), lorsqu'ils ne sont pas connus, peuvent être préparés par un procédé caractérisé en ce que l'on fait réagir un acide anthranilique de formule (VI) :

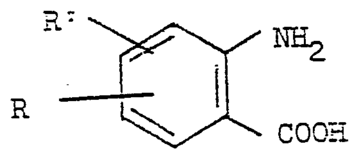

(VI)

dans laquelle R et R' ont la signification déjà indiquée ou un ester d'alcoyle, avec un isothiocyanate de formule (VII) :

$$Y{-}N{=}C{=}S \quad (VII)$$

dans laquelle Y a la signification déjà indiquée, pour obtenir une thioquinazolin-one de formule (VIII) :

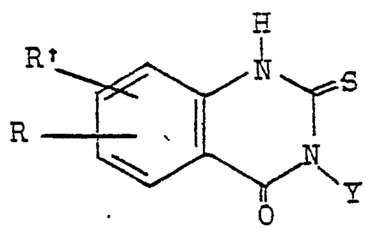

(VIII)

dans laquelle R, R' et Y ont la signification déjà indiquée, puis fait réagir ledit produit avec un hydrate d'hydrazine, pour obtenir le produit de formule (II) recherché.

Certains produits de formule (VIII) sont connus ; il en est ainsi par exemple des produits de formule (VIII) dans laquelle R et R' représentent un atome d'hydrogène, Y un radical méthyle, éthyle ou benzyle (C.A. : *70*,11671 r) ou des produits dans lesquels R et R' représentent un atome d'hydrogène, Y représente un radical allyle ou phényle (C.A. : *61*, 8307 g).

De même, certains produits de formule (II) sont connus ; il en est ainsi par exemple des produits de formule II dans lesquels R et R' représentent un atome d'hydrogène, Y représente un radical méthyle (C.A. : *85*, 5681n) ou R et R' représentent un atome d'hydrogène, Y représente un radical allyle ou phényle.

Certains produits de formule (II), et notamment ceux dans lesquels le substituant Y représente un radical alcoyle, linéaire ou ramifié, renfermant de 1 à 6 atomes de carbone, lorsqu'ils ne sont pas connus, peuvent être préparés par un procédé caractérisé en ce que l'on fait réagir un ester d'acide anthranilique de formule (IX) :

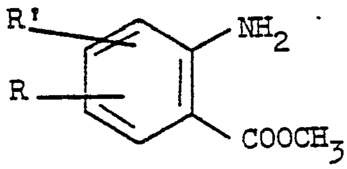

(IX)

dans laquelle R et R' ont la signification déjà indiquée, avec un isocyanate de formule (X) :

$$Y{-}N{=}CO \quad (X)$$

dans laquelle Y a la signification déjà indiquée, pour obtenir un produit de formule (XI) :

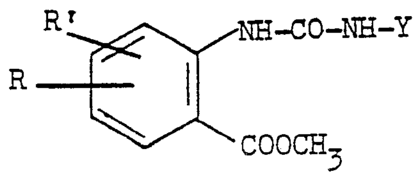

(XI)

dans laquelle R, R' et Y ont la signification déjà indiquée, puis cyclise ce dernier par chauffage en milieu acide pour obtenir un produit de formule (XII) :

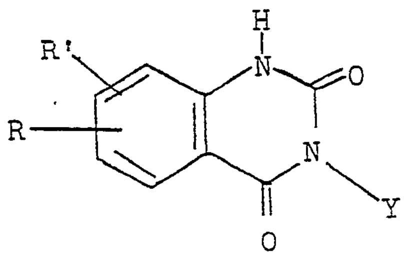

(XII)

dans laquelle R, R' et Y ont la signification déjà indiquée, fait réagir ce dernier avec de l'oxychlorure de phosphore, pour obtenir un produit de formule (XIII) :

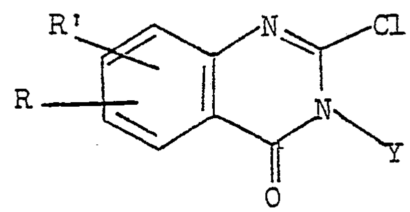

(XIII)

dans laquelle R, R' et Y ont la signification déjà indiquée, puis enfin fait réagir ce dernier avec de l'hydrate d'hydrazine pour obtenir le produit de formule (II) recherché.

Certains produits de formule (XI) sont connus, il en est ainsi par exemple des produits dans lesquels R et R' représentent un atome d'hydrogène, Y représente un radical n-propyle ou n-butyle (C.A. : *56,* 14283b).

De même, certains produits de formule (XII) sont également connus ; il en est ainsi, par exemple des produits dans lesquels R et R' représentent un atome d'hydrogène, Y représente un radical n-propyl ou n-butyl (C.A. : *56,* 14283b), un radical isopropyl (C.A. : *70,* 4141q).

Des exemples de préparation des intermédiaires conduisant aux produits de formule II par l'une des deux voies exposées ci-dessus, sont donnés ci-après dans la partie expérimentale.

Les exemples suivants illustrent l'invention sans toutefois la limiter.

Préparation 1

A) La 2-hydrazino 3-n-propylquinazolin-4 (3H)-one utilisée comme produit de départ de formule (II) dans les exemples 1 à 18 a été préparée comme indiqué ci-dessous (méthode a).

Stade A : N-(N'-n-propylcarbamoyl) anthranilate de méthyle.

On porte au reflux pendant 17 heures, un mélange de 159 g d'anthranilate de méthyle, 5 $cm^3$ de triéthylamine et 134 g d'isocyanate de n-propyle dans 700 $cm^3$ d'éther de pétrole (eb : 60-80 °C). On refroidit ensuite le mélange, filtre le précipité, le lave à l'éther de pétrole et le sèche. On obtient 236 g de produit attendu. F = 101,5°-102,5 °C.

Stade B : 3-n-propylquinazolin-2,4(1H,3H)-dione.

On porte au reflux pendant 3 heures un mélange de 232 g du produit obtenu au stade A et 700 $cm^3$ d'acide chlorhydrique concentré dans 1 litre d'éthanol. On refroidit ensuite le mélange, filtre les cristaux formés, les lave à l'éther et les sèche. On obtient 186 g de produit attendu. F = 188-188,5 °C.

Stade C : 2-chloro 3-n-propylquinazolin-4(3H)-one.

On chauffe au reflux pendant 2 jours, un mélange de 181 g du produit obtenu au stade A et 1 200 $cm^3$ d'oxychlorure de phosphore. On s'assure par chromatographie sur couche mince que le produit de départ a bien totalement disparu. La chromatographie est effectuée de la manière suivante : on prélève un échantillon du mélange réactionnel, le verse dans l'eau, l'y maintient pendant 15 minutes à 0 °C puis

extrait au chloroforme. La chromatographie est effectuée ensuite sur cet extrait. On évapore ensuite l'excès d'oxychlorure de phosphore du mélange réactionnel à moins de 50 °C, verse le mélange résiduel dans un mélange eau-glace sous agitation. On maintient à 0 °C pendant 30 minutes puis extrait à environ 0 °C au chloroforme. On sèche la phase organique, l'évapore à sec et obtient le produit attendu utilisé tel quel pour la suite de la synthèse.

Stade D : 2-hydrazino-3-n-propylquinazolin-4 (3H)-one.

On dissout le produit obtenu au stade C dans 800 $cm^3$ d'éthanol renfermant 400 $cm^3$ d'hydrate d'hydrazine puis le mélange est chauffé au reflux pendant 2 heures. On ajoute 500 $cm^3$ d'eau, refroidit le mélange, filtre les cristaux formés, les lave à l'eau, les sèche et obtient 158 g de produit attendu. F = 125-128 °C.

Spectre IR

Absorption à 761, 1 448, 1 482, 1 568, 1 583, 1 680, 3 320 $cm^{-1}$.

B) En utilisant une méthode analogue à celle utilisée dans la préparation de la 2-hydrazino 3-n-propylquinazolin-4(3H)-one mais en partant du composé de départ correspondant de formule (IX), on obtient la 2-hydrazino 3-isopropylquinazolin-4(3H)-one avec un rendement de 57 %, fondant à 123-124 °C.

Spectre IR

Absorption à 775, 1 315, 1 370, 1 480, 1 585, 1 680, 3 320 $cm^{-1}$.

C) En utilisant une méthode analogue dans celle utilisée dans la préparation du 2-hydrazino 3-n-propylquinazolin-4(3H)-one mais en partant du composé de départ correspondant de formule (IX), on obtient la 3-n-butyl 2-hydrazinoquinazolin-4(3H)-one avec un rendement de 33 %.

Spectre IR

Absorption à 770, 1 060, 1 140, 1 480, 1 570, 1 590, 1 680, 3 290 $cm^{-1}$.

Préparation 2

A) 3-benzyl 2-hydrazinoquinazolin-4(3H)-one, utilisée comme produit de départ de formule (II) dans l'exemple 32, a été préparé comme indiqué ci-dessous (méthode b).

Stade A : 3-benzyl 2-thioquinazolin-2,4(1H, 3H)-dione.

On met en suspension 22,5 g d'acide anthranilique dans 300 $cm^3$ d'éthanol et ajoute 25 g d'isothiocyanate de benzyle. On chauffe le mélange au reflux pendant 4 heures puis refroidit, filtre les cristaux, les lave à l'éther et les sèche et obtient 13,7 g de produit attendu. F = 253-254 °C.

Stade B : 3-benzyl 2-hydrazinoquinazolin-4(3H)-one.

On met en suspension 13,3 g du produit obtenu au stade A dans 160 $cm^3$ d'éthanol puis ajoute 10 $cm^3$ d'hydrate d'hydrazine. On chauffe le mélange au reflux pendant 3 heures, filtre les cristaux formés, les lave à l'éthanol puis à l'éther et les sèche. On obtient 8,4 g de produit. On obtient un second jet de produit en chauffant à nouveau les liqueurs mères, puis en évaporant la solution éthanolique. Les cristaux formés ainsi sont filtrés et séchés. On obtient 1,25 g de produit soit au total 9,65 g. F = 156-158 °C.

B) En utilisant une méthode analogue à celle utilisée dans la préparation de la 3-benzyl 2-hydrazinoquinazolin-4(3H)-one mais en partant du produit de départ correspondant de formule (VI), on a préparé la 3-éthyl 2-hydrazinoquinazolin-4(3H)-one.

Exemple 1 : monohydrate du chlorhydrate de 1-péridinométhyl 4-n-propyl [1,2,4] triazolo [4,3-a] quinazolin 5(4H)-one.

Stade A : 1-chlorométhyl 4-n-propyl [1,2,4] triazolo [4,3-a] quinazolin-5(4H)-one.

On met en suspension 156 g de 2-hydrazino 3-n-propylquinazolin-4(3H)-one dans 500 $cm^3$ de diméthylformamide, puis ajoute lentement en refroidissant au bain de glace 90 g de chlorure de chloroacétyle. On chauffe ensuite le mélange au bain d'eau pendant 3 heures. On verse le mélange réactionnel dans un mélange d'eau, d'acétate d'éthyle et de chlore de méthylène (1-1-0,5). On décante la phase organique, extrait à nouveau la phase aqueuse à l'acétate d'éthyle. On réunit les phases

organiques, les lave à l'eau, les sèche et évapore le solvant. On triture le résidu dans l'éther, le lave à l'éther et le sèche. On obtient 146 g de produit attendu. F = 141-145 °C.

Stade B : Monohydrate du chlorhydrate de 1-pipéridinométhyl-4-n-propyl [1,2,4] triazolo [4,3-a] quinazolin-5(4H)-one.

On met en suspension 111 g de produit obtenu au stade A, dans 500 $cm^3$ de toluène puis ajoute 73,6 g de pipéridine. On chauffe le mélange au reflux pendant 2 heures, verse dans un mélange d'eau et d'acétate d'éthyle (1-1), décante, extrait à nouveau la phase aqueuse à l'acétate d'éthyle. On réunit les phases organiques, les lave à l'eau, les sèche et évapore le solvant. On obtient un produit cristallisé que l'on triture dans l'éther, sèche. On obtient 108,6 g de produit attendu. F = 160-166 °C.

Conversion du produit obtenu ci-dessus en hydrate du chlorhydrate.

On dissout 11 g de produit obtenu ci-dessus dans 500 $cm^3$ d'éthanol puis ajoute un mélange d'acide chlorhydrique dans l'éther puis de l'éther. On filtre les cristaux formés et les sèche. On obtient 84,4 g de produit attendu. Un second jet de 20 g de produit attendu a été obtenu à partir de liqueurs mères, puis l'ensemble des cristaux formés (104,4 g) est recristallisé dans l'éthanol par addition d'éther. On filtre les cristaux, les lave à l'éther et les sèche à 70 °C. On laisse ensuite le produit s'hydrater à l'air. On obtient finalement 100,6 g de produit attendu. F = 210-216 °C.

Exemples 2 à 42

Stade A

En utilisant une méthode analogue à celle décrite au stade A de l'exemple 1 et en partant du produit de départ correspondant de formule (II), on prépare les produits suivants de formule (IV) dans laquelle Hal représente un atome de chlore, comme indiqué dans le tableau ci-dessous. Dans les tableaux I et II ci-dessous, « méthode a » indique que le produit correspondant de formule (II) a été préparé à partir d'un produit de formule (IX) comme décrit dans la préparation 1 et « méthode b » indique que le produit correspondant de formule (II) a été préparé à partir d'un produit de formule (VI) comme décrit dans la préparation 2. La 4-allyl 1-(2-chloroéthyl) [1,2,4] triazolo [4,3-a] quinazolin-5(4H)-one préparée au stade A de l'exemple 35, a été également préparée comme indiqué ci-dessous, cette préparation étant indiquée dans le tableau 1 comme étant la « méthode d ».

Stade A : 3-allyl 2-[2-(3-chloropropionyl) hydrazino] quinazolin-4(3H)-one.

On ajoute à un mélange de 4,50 g de 3-allyl 2-hydrazinoquinazolin-4(3H)-one et 5,75 g de carbonate de potassium anhydre dans 100 $cm^3$ de chloroforme, à température ambiante, 2,91 g de chlorure de 3-chloropropionyl. Un précipité apparaît immédiatement et après 1 heure, on ajoute 50 $cm^3$ d'eau sous agitation pour dissoudre le carbonate de potassium. On filtre, lave les cristaux formés à l'eau puis à l'éther, les recristallise dans l'éthanol, les sèche à 80 °C et obtient 5,79 g de produit attendu. F = 151-153 °C.

Stade B : 4-allyl 1-(2-chloroéthyl) [1,2,4] triazolo [4,3-a] quinazolin-5(4H)-one.

On porte au reflux pendant 17 heures un mélange de 5,3 g de produit obtenu au stade A, et 10 mg d'hydrade d'acide paratoluène sulfonique dans 300 $cm^3$ d'éthanol. On évapore ensuite le solvant, dissout l'huile résiduelle dans le chlorure de méthylène, lave à l'eau, sèche et évapore à sec. On purifie le produit obtenu par chromatographie sur silice en éluant au chlorure de méthylène à 1 % de méthanol. On recristallise le produit obtenu dans l'éthanol. On obtient 2,625 g de produit attendu. F = 142,5-144 °C.

(Voir Tableau I pages 10 et 11)

Tableau I

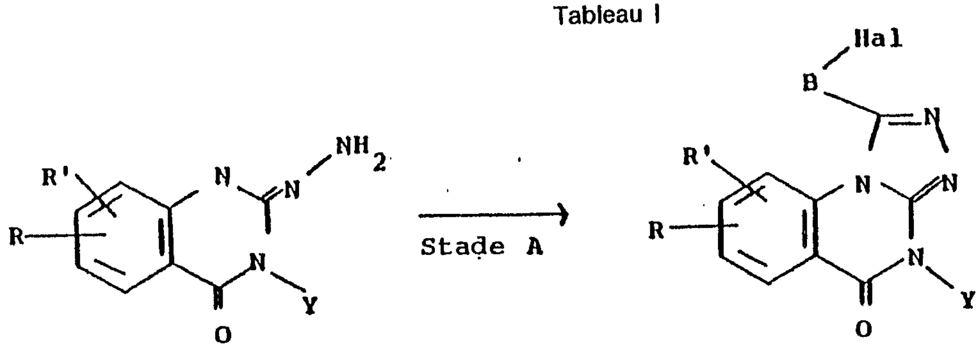


(IV) (II) (Hal=Cl)

| Ex | R | R' | B | Y | méthode | Rendement % | Solvant de recristallisation | IR $cm^{-1}$ |
|---|---|---|---|---|---|---|---|---|
| 1-18 | H | H | $CH_2$ | $-(CH_2)_2-CH_3$ | a | 74 | EtOAc | 756,1497,1569,1618,1686 |
| 19-20 | H | H | $CH_2$ | $-CH(CH_3)_2$ | a | 65 | EtOAc | 765,1275,1460,1500,1570, 1610,1623,1695 |
| 21-22 | H | H | $CH_2$ | $-(CH_2)_3-CH_3$ | b | 67 | EtOAc | 760,1300,1500,1570,1605,1620, 1695 |
| 23-28, 36,37 | H | H | $CH_2$ | $-CH_3$ | b | 52 | EtOAc/$CHCl_3$ | 760,1500,1567,1603,1621, 1682 |
| 29-30 | H | H | $CH_2$ | $-C_2H_5$ | b | 78 | EtOAc | |
| 31 | H | H | $CH_2$ | 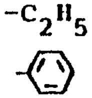 | b | 43 | EtOAc/$Et_2O$ | 751,1298,1490,1552,1691 |
| 32 | H | H | $CH_2$ | $-CH_2-$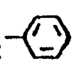 | b | 79 | EtOAc/$Et_2O$ | |
| 33-34 | H | H | $CH_2$ | allyl | b | 78 | EtOAc/$Et_2O$ | 765,1490,1565,1600,1615, 1685 |
| 35 | H | H | $(CH_2)_2$ | allyl | b+d | 53 | EtOH | 755,1498,1574,1615,1675 |

Tableau I (Suite)

| Ex | R | R' | B | Y | méthode | Rendement % | Solvant de recristallisation | IR $cm^{-1}$ |
|---|---|---|---|---|---|---|---|---|
| 38 | 8-Cl | H | $CH_2$ | $-(CH_2)_3CH_3$ | b | 70 | EtOAc | 848, 1292, 1494, 1557, 1591, 1612, 1690 |
| 39 | 7-Cl | H | $CH_3$ | $-(CH_2)_3CH_3$ | a | 69 | EtOAc/$Et_2O$ | 815, 1487, 1563, 1605, 1694 |
| 40 | 8-Cl | H | $CH_2$ | $-(CH_2)_2CH_3$ | a | 75 | EtOAc/$Et_2O$ | 850, 1495, 1592, 1613, 1690 |
| 41 | 7-Cl | H | $CH_2$ | $-(CH_2)_2CH_3$ | a | 71 | EtOAc | 810, 1254, 1490, 1559, 1593 1605, 1685 |
| 42 | 7-Me | H | $CH_2$ | $-(CH_2)_3CH_3$ | b | 65 | EtOAc | 809, 1503, 1566, 1686 |

Stade B

En utilisant une méthode analogue à celle utilisée au stade B de l'exemple 1 et en utilisant au départ des composés correspondants de formule (IV), on obtient les produits de formule (I) figurant aux exemples 1 à 35 et 38 à 42 comme indiqué dans le tableau II ci-dessous.

Le produit de l'exemple 37 a été préparé comme indiqué ci-après.

On chauffe au reflux pendant 16 heures un mélange de 1,5 g de 1-chlorométhyl 4-méthyl [1.2.4] triazolo [4,3-a] quinazolin-5(4H)-one et 0,7 g d'imidazolidinethione dans 30 $cm^3$ d'acétone. Le produit cristallisé formé est filtré, lavé à l'acétone et séché. On obtient 1,95 g de produit (F = 245-250 °C avec décomposition), que l'on recristallise dans un mélange méthanol-éther.

Le produit de l'exemple 36 a été préparé de manière analogue à celle du produit de l'exemple 37.

Les microanalyses des composés de formule (I) préparés dans les exemples 1 à 42, sont donnés dans le tableau III ci-après.

(Voir Tableaux II et III pages 13 à 19)

Exemple 43 : Monohydrate du chlorhydrate de 1-pipéridinométhyl 4-n-propyl [1,2,4] triazolo [4,3-a] quinazolin-5(4H)-one.

On met en suspension de 5 g de 4-allyl 1-piridinométhyl [1,2,4] triazolo [4,3-a] quinazolin-5(4H)-one (préparé dans l'exemple 33) dans 300 $cm^3$ d'un mélange chloroforme-éthanol (1-1) et ajoute 500 mg de palladium à 5 % sur charbon. On maintient le mélange résultant pendant 1 heure et demie sous hydrogène jusqu'à absorption de la quantité théorique d'hydrogène. On filtre le mélange, évapore le solvant et obtient 4,3 g de produit attendu brut. On transforme ce produit en monohydrate du chlorhydrate comme décrit dans l'exemple 1. Le produit ainsi obtenu a le même point de fusion que le produit obtenu à l'exemple 1.

Exemples de compositions pharmaceutiques.

Exemple 44

On a préparé des comprimés ayant la formulation suivante :

| | |
|---|---|
| Produit de l'exemple 1 | 15 mg |
| Excipient q.s. pour un comprimé | 100 mg |

(Détail de l'excipient : lactose, talc, amidon, stéarate de magnésium).

Exemple 45

On a préparé des aérosols délivrant par dose :

| | |
|---|---|
| Produit de l'exemple 1 | 2 mg |
| Emulsifiant | 0,15 mg |
| Propulseur | 50 mg |

Exemple 46

On a préparé un sirop ayant la formulation suivante :

| | |
|---|---|
| Produit de l'exemple 1 | 0,3 mg |
| Excipient aromatisant q.s.p. | 100 ml. |

Etude pharmacologique

A) Action sur le bronchospasme provoqué par l'histamine chez le cobaye (test A).

On opère suivant la méthode de Konzett et Rossler (Arch. exp. Path. Pharmakol. *1195*. 71 (1940)).

On utilise des cobayes mâles Dunkin Hartley pesant de 350 à 650 g, anesthésiés par voie intrapéritonéale par de l'uréthane (7 ml/kg d'une solution à 25 % dans l'eau distillée).

On opère comme décrit par Konzett et Rossler, à l'aide d'une pompe injectant des doses d'air de 8 $cm^3$ à raison de 52 injections par minutes, afin de maintenir une surpression dans les poumons d'environ 10 cm d'eau.

Tableau II

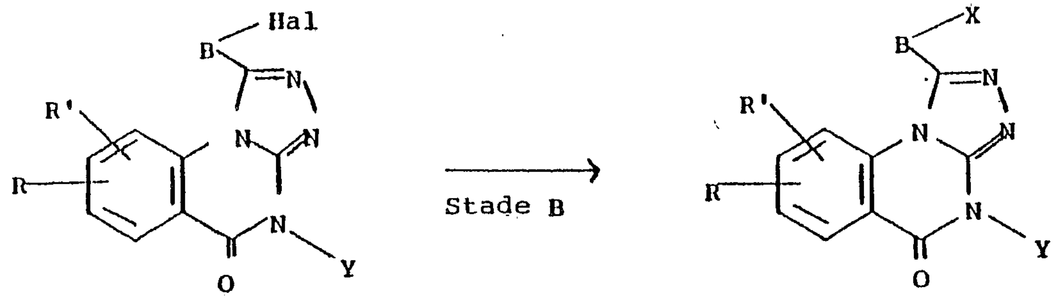


(II) (Hal=Cl) (I)

| Ex | R | R' | B | Y | X | Rend. % | Solvant de recristallisation | IR $cm^{-1}$ |
|---|---|---|---|---|---|---|---|---|
| 1 | H | H | $CH_2$ | $-(CH_2)_2-CH_3$ | 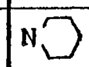 .HCl | 67 | $MeOH/Et_2O$ | 761,1503,1587,1623,1696, 2400-2700,3300-3600 |
| 2 | H | H | $CH_2$ | $-(CH_2)_2-CH_3$ | $NEt_2$.HCl | 61 | $EtOH/Et_2O$ | 759,1420,1578,1618,1690, 2200-2600,3300-3600 |
| 3 | H | H | $CH_2$ | $-(CH_2)_2-CH_3$ | 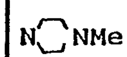NMe.2HCl | 46 | $EtOH/Et_2O$ | 757,1499,1571,1692, 2300-2700,3200-3600 |
| 4 | H | H | $CH_2$ | $-(CH_2)_2-CH_3$ | 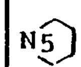 .HCl | 64 | $EtOH/Et_2O$ | 758,1497,1576,1685, 2300-2700,3100-3600 |
| 5 | H | H | $CH_2$ | $-(CH_2)_2-CH_3$ | 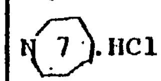.HCl | 67 | $EtOH/Et_2O$ | 758,1498,1572,1700, 2400-2700,3200-3650 |
| 6 | H | H | $CH_2$ | $-(CH_2)_2-CH_3$ | 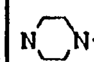N-Ph.HCl | 51 | $EtOH/Et_2O$ | 755,1496,1566,1679, 2300-2600,3300-3600 |
| 7 | H | H | $CH_2$ | $-(CH_2)_2-CH_3$ | 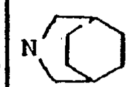 .HCl | 42 | $EtOH/Et_2O$ | 756,1496,1572,1699, 2500-2800 |
| 8 | H | H | $CH_2$ | $-(CH_2)_2-CH_3$ | 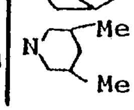 .HCl | 50 | $EtOH/Et_2O$ | 757,1070,1512,1620,1663, 1723,2000-2700,3300-3600 |

Tableau II (Suite)

| Ex | R | R' | B | Y | X | Rendt. % | Solvant de recristallisation | IR $cm^{-1}$ |
|---|---|---|---|---|---|---|---|---|
| 9 | H | H | $CH_2$ | $-(CH_2)_2CH_3$ | 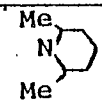 .HCl | 20 | $EtOH/Et_2O$ | 751,1462,1511,1576,1680, 2000-2500 |
| 10 | H | H | $CH_2$ | $-(CH_2)_2CH_3$ | NH nPr.HCl | 74 | $EtOH/Et_2O$ | 765,1280,1515,1580,1620, 1670,1730,2360-2840, 3380-3560 |
| 11 | H | H | $CH_2$ | $-(CH_2)_2CH_3$ | 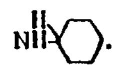.HCl | 79 | $EtOH/Et_2O$ | 760,1505,1585,1625,1665, 1705,1730,2400-2900, 3300-3600 |
| 12 | H | H | $CH_2$ | $-(CH_2)_2CH_3$ | NH nBu.HCl | 75 | $EtOH/Et_2O$ | 760,1505,1580,1605,1625, 1702,2500-2850,3320-3600 |
| 13 | H | H | $CH_2$ | $-(CH_2)CH_3$ | $N(isoPr)_2$.HCl | 45 | $EtOH/Et_2O$ | 751,1505,1583,1600,1615, 1690,2400-2700,3200-3650 |
| 14 | H | H | $CH_2$ | $-(CH_2)_2CH_3$ | $N(nBu)_2$.HCl | 61 | $EtOH/Et_2O$ | 760,1505,1575,1605,1625, 1690,2300-2500,3300-3500 |
| 15 | H | H | $CH_2$ | $-(CH_2)_2CH_3$ | $N(isoBu)_2$.HCl | | $EtOH/Et_2O$ | 756,1266,1503,1574,1600, 1615,1690,2360-2800,3300-3600 |
| 16 | H | H | $CH_2$ | $-(CH_2)_2CH_3$ | $NHCH_2CH_2$-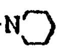. 2HCl | 79 | $EtOH/Et_2O$ | 765,1290,1590,1625,1670, 1695,1725,2500-2800, 3300-3550 |
| 17 | H | H | $CH_2$ | $-(CH_2)_2CH_3$ | $NMe_2$.HCl | 76 | $EtOH/Et_2O$ | 765,1370,1480,1515,1575, 1615,1665,1725 |
| 18 | H | H | $CH_2$ | $-(CH_2)_2CH_3$ | 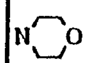 2HCl | 73 | $EtOH/Et_2O$ | 760,1075,1515,1610,1645, 1700,2000-2500,3300-3550 |
| 19 | H | H | $CH_2$ | 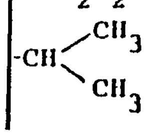 | 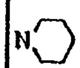 .HCl | 65 | $EtOH/Et_2O$ | 760,1285,1460,1500,1570, 1620,1650,1705,2100-2700, 3300-3550 |

Tableau II (Suite)

| Ex | R | R' | B | Y | X | Rendt. % | Solvant de recristallisation | IR $cm^{-1}$ |
|---|---|---|---|---|---|---|---|---|
| 20 | H | H | $CH_2$ | $-CH(CH_3)CH_3$ | 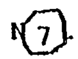.HCl | 71 | $EtOH/Et_2O$ | 765,1295,1420,1495,1575, 1605,1620,1700,2500-2700, 3300-3550 |
| 21 | H | H | $CH_2$ | $-(CH_2)_3CH_3$ | 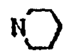 | 72 | $EtOH/Et_2O$ | 761,1281,1612,1712,2200-2750,3250-3600 |
| 22 | H | H | $CH_2$ | $-(CH_2)_3CH_3$ | 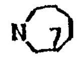 .HCl | 60 | $EtOH/Et_2O$ | 765,1080,1285,1580,1620, 1655,1720,2200-2700, 3200-3550 |
| 23 | H | H | $CH_2$ | $-CH_3$ | 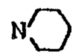 HCl | 64 | $EtOH/Et_2O$ | 760,1507,1573,1608,1622, 1692,2350-2700,3200-3600 |
| 24 | H | H | $CH_2$ | $-CH_3$ | 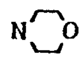O.HCl | 73 | $EtOH/Et_2O$ | 759,1505,1572,1621,1701, 2350-2700,3200-3600 |
| 25 | H | H | $CH_2$ | $-CH_3$ | 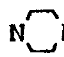$NCO_2Et$.2HCl | 70 | $EtOH/Et_2O$ | 760,1238,1434,1486,1575, 1618,1690,2100-2500, 3200-3550 |
| 26 | H | H | $CH_2$ | $-CH_3$ | $NEt_2$.HCl | 63 | $EtOH/Et_2O$ | 759,1503,1574,1620,1683, 2400-2750,3300-3600 |
| 27 | H | H | $CH_2$ | $-CH_3$ | 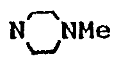NMe.HCl | 68 | $EtOH/Et_2O$ | 760,1506,1618,1676,2350-2750,3200-3600 |
| 28 | H | H | $CH_2$ | $-CH_3$ | 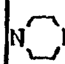$NCH_2CH_2OH$.HCl | 70 | $EtOH/Et_2O$ | 754,1294,1618,1673,1718, 2200-2700,3200-3600 |
| 29 | H | H | $CH_2$ | $-C_2H_5$ | 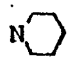 2HCl | 75 | $EtOH/Et_2O$ | |
| 30 | H | H | $CH_2$ | $-C_2H_5$ | $NEt_2$ 2HCl | 67 | $EtOH/Et_2O$ | |
| 31 | H | H | $CH_2$ | 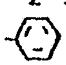 | 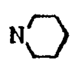 .HCl | 86 | $EtOH/Et_2O$ | 760,1500,1556,1698,2400-2700,3200-3600 |

Tableau II (Suite)

| Ex | R | R' | B | Y | X | Rendt. % | Solvant de recristallisation | IR $cm^{-1}$ |
|---|---|---|---|---|---|---|---|---|
| 32 | H | H | $CH_2$ | $-CH_2-$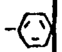 | 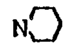 .HCl | 75 | $EtOH/Et_2O$ | 760,1500,1556,1696, 2350-2700,3300-3500 |
| 33 | H | H | $CH_2$ | allyl | 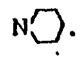 .HCl | 53 | $EtOH/Et_2O$ | 757,1271,1490,1574,1600, 1615,1690,2360-2700,2950, 3300-3620 |
| 34 | H | H | $CH_2$ | allyl | 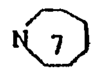 .HCl | 79 | $EtOH/Et_2O$ | 755,1495,1570,1595,1615, 1690,2500-2750,3300-3600 |
| 35 | H | H | $(CH_2)_2$ | allyl | 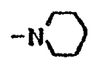 .HCl | 32 | $EtOH/Et_2O$ | 755,1493,1593,1598,1615, 1704,2500-2750,3200-3700 |
| 36 | H | H | $CH_2$ | $-CH_3$ | 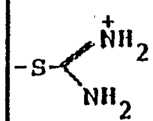 $Cl^-$ | 72 | $Et_2CO$ | 760,1502,1580,1625,1690, 2600-3550 |
| 37 | H | H | $CH_2$ | $-CH_3$ | 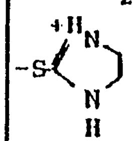 $Cl^-$ | 92 | $EtOH/Et_2O$ | 760,1500,1576,1596,1621, 1697,2600-3500 |
| 38 | 8-Cl | H | $CH_2$ | $-(CH_2)_3CH_3$ | 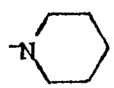 HCl | 77 | EtOH | 1478,1592,1610,1687,2360-2660br, 3340-3600br |
| 39 | 7-Cl | H | $CH_2$ | $-(CH_2)_3CH_3$ | 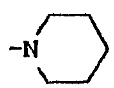 HCl | 83 | $EtOH/Et_2O$ | 816,1240,1486,1560,1608, 1695,2700-2240br,3660-3330br |
| 40 | 8-Cl | H | $CH_2$ | $-(CH_2)_2CH_3$ | 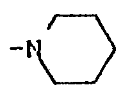 HCl | 77 | EtOH | 1475,1590,1610,1683,2360-2660br, 3300-3600br |
| 41 | 7-Cl | H | $CH_2$ | $-(CH_2)_2CH_3$ | 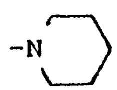 HCl | 58 | $EtOH/Et_2O$ | 815,1484,1570,1598,1610,1690, 2200-2500br,3300-3600br |
| 42 | 7-Me | H | $CH_2$ | $-(CH_2)_3CH_3$ | 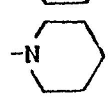 HCl | 61 | $EtOH/Et_2O$ | 1418,1603,1654,1711,2200-2750br, 3300-3500br |

Tableau III

| Exemples | F. °C | Formule | P.Mol. (incl. $H_2O$) | moles $H_2O$ | ANALYSES CALCULE %C | %H | %N | %Cl | TROUVE %C | %H | %N | %Cl |
|---|---|---|---|---|---|---|---|---|---|---|---|---|
| 1 | 211-14 | $C_{18}H_{24}N_5OCl$ | 379.9 | 1.0 | 56.91 | 6.90 | 18.43 | 9.93 | 56.90 | 6.90 | 18.62 | 9.63 |
| 2 | 178-82 | $C_{17}H_{24}N_5OCl$ | 358.9 | 0.5 | 56.90 | 7.02 | 19.51 | 9.88 | 56.97 | 6.99 | 19.66 | 10.25 |
| 3 | 225-30 | $C_{18}H_{26}N_6OCl_2$ | 462.9 | 2.75 | 46.71 | 6.86 | 18.16 | 15.32 | 46.76 | 6.84 | 18.33 | 15.43 |
| 4 | 228-32 | $C_{17}H_{22}N_5OCl$ | 365.9 | 1.0 | 55.81 | 6.61 | 19.14 | 9.69 | 55.29 | 6.66 | 18.97 | 9.64 |
| 5 | 196-200 | $C_{19}H_{26}N_5OCl$ | 393.9 | 1.0 | 57,93 | 7.16 | 17.78 | 9.00 | 57.92 | 7.17 | 17.89 | 8.98 |
| 6 | 225-32 | $C_{23}H_{27}N_6OCl$ | 452.5 | 0.75 | 61,06 | 6.35 | 18.57 | 7.83 | 61.03 | 6.54 | 18.26 | 7.54 |
| 7 | 212-6 | $C_{21}H_{28}N_5OCl$ | 401.9 | - | 62.75 | 7.02 | 17.42 | 8.82 | 62.71 | 7.01 | 17.38 | 8.92 |
| 8 | 215-20 | $C_{20}H_{28}N_5OCl$ | | | | | | | | | | |
| 9 | 228-30 | $C_{20}H_{28}N_5OCl$ | | | | | | | | | | |
| 10 | 225-228 | $C_{16}H_{22}N_5OCl$ | 335,8 | - | 57,22 | 6.60 | 20.85 | 10.56 | 57,20 | 6,62 | 20.85 | 10.56 |
| 11 | 210-215 | $C_{19}H_{26}N_5OCl$ | 403,4 | 0.75 | 59.54 | 7.37 | 17.36 | 8.79 | 59.52 | 7.39 | 17.24 | 8.92 |
| 12 | 216-219 | $C_{17}H_{24}N_5OCl$ | 367,9 | 1.0 | 55.50 | 7.12 | 19.04 | 9.64 | 55.21 | 7.09 | 18.88 | 9.55 |
| 13 | 158-163 | $C_{19}H_{28}N_5OCl$ | 377.9 | - | 60.39 | 7.47 | 18.53 | 9.38 | 60.54 | 7.50 | 18.62 | 9.46 |
| 14 | 150-154 | $C_{21}H_{32}N_5OCl$ | | | | | | | | | | |
| 15 | 112-17 | $C_{21}H_{32}N_5OCl$ | 406.0 | - | 62,13 | 7.95 | 17.25 | 8.73 | 61.89 | 7.90 | 17.41 | 8.77 |
| 16 | 141-44 | $C_{20}H_{30}N_6OCl_2$ | 477.4 | 2.0 | 50.31 | 7.18 | 17.60 | | 50.03 | 7.24 | 17.55 | |
| 17 | 203-06 | $C_{15}H_{20}N_5OCl$ | 344.3 | 1,25 | 52,32 | 6.59 | 20.34 | 10.30 | 52.34 | 6.53 | 20.28 | 10.37 |
| 18 | 199-202 | $C_{17}H_{23}N_5O_2Cl_2$ | 400.3 | - | 51.01 | 5.79 | 17.49 | 17.71 | 51.03 | 5.84 | 17.42 | 17.36 |

Tableau III (Suite)

| Exemples | F. °C | Formule | P.Mol. (incl. $H_2O$) | moles $H_2O$ | CALCULE %C | CALCULE %H | CALCULE %N | CALCULE %Cl | TROUVE %C | TROUVE %H | TROUVE %N | TROUVE %Cl |
|---|---|---|---|---|---|---|---|---|---|---|---|---|
| 19 | 151-154 | $C_{18}H_{24}H_5OCl$ | 379.9 | 1.0 | 56.91 | 6.90 | 18.43 | 9.33 | 57.17 | 6.85 | 18.58 | 9.40 |
| 20 | 144-145.5 | $C_{19}H_{26}N_5OCl$ | 389.4 | 0.75 | 58,60 | 7.12 | 17.98 | 9.10 | 58.51 | 7.21 | 17.41 | 9.39 |
| 21 | 156-156.5 | $C_{19}H_{26}N_5OCl$ | 339.4 | - | 67.23 | 7.42 | 20.63 | | 67.29 | 7.41 | 20.67 | |
| 22 | 122-126 | $C_{20}H_{29}N_5OCl_2$ | 439.9 | 0.75 | 54.61 | 6,99 | 15.92 | 16.12 | 54.68 | 6.89 | 15.94 | 15.72 |
| 23 | 236-40 | $C_{16}H_{20}N_5OCl$ | 338.3 | 0.25 | 56.80 | 6.11 | 20.70 | 10.48 | 56.85 | 6.07 | 20.59 | 10.65 |
| 24 | 225-28 | $C_{15}H_{18}N_5O_2Cl$ | 335.8 | - | 53.65 | 5.40 | 20.86 | 10.56 | 53.54 | 5.40 | 20.67 | 10.44 |
| 25 | 175-80 | $C_{18}H_{24}N_6O_3Cl_2$ | 461.4 | 1.0 | 46,85 | 5.64 | 18.22 | 15.40 | 46.66 | 5.61 | 18.16 | 15.38 |
| 26 | 210-12 | $C_{15}H_{20}N_5OCl$ | 321.8 | - | 55.99 | 6.22 | 21.77 | 11.04 | 55.76 | 6.28 | 21.54 | 10.94 |
| 27 | 290-92 | $C_{16}H_{22}N_6OCl$ | 366.9 | 1.0 | 52.38 | 6.32 | 22.91 | 9.66 | 52.20 | 6.02 | 22.75 | 9.87 |
| 28 | 301-03 | $C_{17}H_{24}N_6O_2Cl_2$ | 396.9 | 1.0 | 51.45 | 6.35 | 21.18 | 8.93 | 51.25 | 6.29 | 21.21 | 9.07 |
| 29 | 180-85 | $C_{17}H_{23}N_5OCl_2$ | 402.3 | 1.0 | 50.76 | 6.26 | 17.41 | 17.63 | 50.97 | 6.12 | 17.35 | 17.53 |
| 30 | 158-60 | $C_{16}H_{23}N_5OCl_2$ | 381.3 | 0.5 | 50.40 | 6.08 | 18.60 | 18.37 | 50.03 | 6.18 | 18.27 | 18.03 |
| 31 | 233-38 | $C_{23}H_{28}N_5OCl$ | 441.0 | 1.0 $C_2H_5OH$ | 62.51 | 6.39 | 15.85 | 8.02 | 62.41 | 6.33 | 15.85 | 8.20 |
| 32 | 223-36 | $C_{24}H_{30}N_5OCl$ | 456.0 | 1.0 $C_2H_5OH$ | 63.22 | 6.63 | 15.36 | 7.77 | 63.00 | 6.47 | 15.47 | 7.86 |
| 33 | 208-209 | $C_{18}H_{22}N_5OCl$ | 377.9 | 1.0 | 57.22 | 6,40 | 18.53 | 9.38 | 57.08 | 6.28 | 18.79 | 9.51 |
| 34 | 172-74 | $C_{19}H_{24}N_5OCl$ | 391.9 | 1.0 | 58,23 | 6.69 | 17.87 | 9.05 | 58.35 | 6.73 | 18.04 | 9.32 |
| 35 | 228-34 | $C_{19}H_{24}N_5OCl$ | 391.9 | 1.0 | 58,23 | 6.69 | 17.87 | 9.05 | 58.54 | 6.65 | 17.77 | 9.14 |
| 36 | 227-30 | $C_{12}H_{13}N_6SOCl$ | 324.8 | 1.0 | 42.04 | 4.38 | 24.53 | 10.36 | 42.43 | 4.29 | 24.61 | 10.44 |
| 37 | 245-50 | $C_{14}H_{15}N_6OSCl$ | 377.9 | 1.5 | 44.50 | 4.80 | 22.24 | 9.38 | 44.71 | 4.51 | 22.23 | 9.38 |

Tableau III (Suite)

| Exemples | F. °C | Formule | P.Mol. (incl. $H_2O$) | moles $H_2O$ | CALCULE | | | | TROUVE | | | |
|---|---|---|---|---|---|---|---|---|---|---|---|---|
| | | | | | %C | %H | %N | %Cl | %C | %H | %N | %Cl |
| 38 | 188-95 | $C_{19}H_{25}N_5OCl_2$ | 410.4 | | 55.61 | 6.14 | 17.07 | 17.28 | 55.74 | 6.10 | 17.27 | 17.21 |
| 39 | 193-200 | $C_{19}H_{25}N_5OCl_2$ | 410.4 | | 55.61 | 6.14 | 17.07 | 17.28 | 55.58 | 6.10 | 17.23 | 17.49 |
| 40 | 281-87 | $C_{18}H_{23}N_5OCl_2$ | 414.1 | 1.0 | 52.18 | 6.09 | 16.90 | 17.11 | 52.26 | 6.04 | 17.12 | 17.19 |
| 41 | 200-01 | $C_{18}H_{25}N_5OCl_2$ | 396.3 | | 54.55 | 5.85 | 17.67 | 17.89 | 54.60 | 5.84 | 17.79 | 17.90 |
| 42 | 227-29 | $C_{20}H_{29}N_5OCl_2$ | 444.4 | 1.0 | 54.05 | 7.03 | 15.75 | 15.96 | 54.05 | 6.89 | 15.83 | 15.90 |

On mesure la pression sanguine dans l'artère carotide droite.

La substance à tester est administrée par la veine jugulaire gauche, le rinçage du dispositif étant assuré par 0,1 $cm^3$ d'une solution à 0,9 % de chlorure de sodium dans l'eau distillée. La substance à tester est administrée immédiatement avant l'histamine qui est utilisée comme agoniste.

On enregistre les variations respiratoires.

Les résultats figurant dans le tableau ci-après expriment la dose efficace de chaque produit, requise pour réduire de 50 % la constriction induite par l'histamine.

B) Mesure de la résistance respiratoire (test B).

On utilise des cobayes mâles Dunkin-Hartley pesant de 400 à 650 g, anesthésiés par une combinaison d'Hypnorm (1 ml/kg i.m.) et de Valium (5 mg/kg i.p.) et dont la trachée est intubée.

La substance à tester est administrée par la veine jugulaire droite et la pression sanguine est mesurée dans l'artère carotide gauche.

Les animaux sont ventilés à raison de 6 à 8 $cm^3$ d'air par injection et une différence de pression de 10 à 12 cm d'eau est obtenue. 40 à 50 injections d'air par minute sont réalisées.

Les animaux sont traités au départ par voie intraveineuse par une dose de 4 mg/kg, de manière à obtenir une paralysie des muscles respiratoires.

L'air injecté est enrichi en oxygène.

La résistance respiratoire est mesurée par la méthode de Clay et Hughes. J. Physiol. 308, 427-427 (1980).

L'administration du produit à tester et l'analyse des résultats sont effectuées comme décrit au test A.

Les résultats figurant dans le tableau ci-après, montrent la dose efficace de chaque produit, nécessaire pour réduire de 50 % la résistance induite par l'histamine.

| Produit de l'exemple | Test A $DE_{50}$ (mg/kg) | Test B $DE_{50}$ (mg/kg) |
|---|---|---|
| 1 | 0,12 | |
| 2 | | 0,50 |
| 3 | 5,28 | |
| 4 | 2,0 | |
| 5 | 0,096 | 0,3 |
| 6 | 5,0 | |
| 7 | 0,33 | |
| 8 | 1,0 | |
| 10 | 1,97 | |
| 11 | 1,8 | |
| 12 | 1,3 | |
| 14 | 2,9 | |
| 19 | 0,185 | |
| 20 | 0,8 | |
| 21 | 0,11 | 0,15 |
| 22 | 0,84 | |
| 23 | >1 | 1,0 |
| 24 | 2,76 | 1,8 |
| 25 | 5,0 | 3,5 |
| 26 | 3,75 | 1,0 |
| 27 | | 0,8 |
| 29 | 0,35 | 0,5 |
| 30 | | 1,0 |
| 31 | 0,58 | |
| 33 | 0,31 | |

**Revendications** (pour les Etats contractants : BE, CH, DE, FR, IT, LI, LU, NL, SE)

1. Nouvelles triazoloquinazolones, ainsi que leurs sels, caractérisées en ce qu'elle répondent à la formule générale (I) :

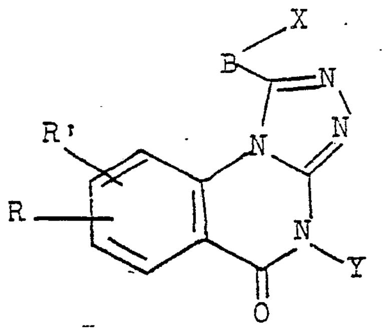

(I)

dans laquelle

R et R', identiques ou différents, représentent un atome d'hydrogène, un atome d'halogène, un radical alcoyle renfermant de 1 à 3 atomes de carbone, un radical alcoxy renfermant de 1 à 3 atomes de carbone, un radical nitro,

Y représente un radical alcoyle, linéaire ou ramifié, renfermant de 1 à 6 atomes de carbone, un radical cycloalcoyle renfermant de 3 à 8 atomes de carbone, un radical alcényle renfermant de 2 à 4 atomes de carbone, un radical aryle renfermant de 6 à 8 atomes de carbone ou aralcoyle renfermant 7 à 8 atomes de carbone,

B représente un groupement

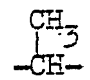

ou un radical alcoylène $-(CH_2)n-$ dans lequel n représente un nombre entier égal à 1, 2 ou 3,

X représente un groupement

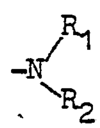

dans lequel $R_1$ et $R_2$, identiques ou différents, représentent un atome d'hydrogène, un groupement alcoyle, linéaire ou ramifié, renfermant de 1 à 6 atomes de carbone, un radical cycloalcoyle renfermant de 3 à 8 atomes de carbone, un radical aryle ou aralcoyle renfermant de 6 à 8 atomes de carbone, un radical aminoalcoyle, monoalcoyl ou dialcoylaminoalcoyle dans lesquels les groupements alcoyles renfermant de 2 à 4 atomes de carbone, un radical pipéridinoalcoyle, morpholino-alcoyle ou pipérazinylalcoyle ou $R_1$ et $R_2$ forment ensemble avec l'atome d'azote auxquels ils sont liés un hétérocycle saturé mono ou bicyclique renfermant de 4 à 8 atomes de carbone pouvant être substitué par 1 ou 2 radicaux méthyle, ou pouvant renfermer en outre un atome d'oxygène, de soufre et d'azote, ledit atome d'azote pouvant être substitué par un radical alcoyle renfermant de 1 à 3 atomes de carbone, par un radical hydroxyalcoyle renfermant de 1 à 3 atomes de carbone, par un radical aryle ou aralcoyle renfermant de 6 à 8 atomes de carbone, ou par un radical alcoxycarbonyle renfermant de 2 à 5 atomes de carbone, ou enfin, X représente un radical

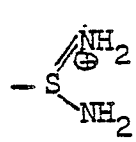 Cl ⊖ ou 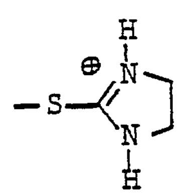 Cl ⊖

2. Dérivés répondant à la formule générale (I) de la revendication 1, ainsi que leurs sels, caractérisés en ce que dans ladite formule (I),

R et R', identiques ou différents, représentent un atome d'hydrogène, un atome de chlore, un radical méthyle, un radical méthoxy ou un radical nitro,

Y représente un radical alcoyle, linéaire ou ramifié, renfermant de 1 à 6 atomes de carbone, un radical cyclohexyle, un radical allyle, un radical phényle ou benzyle,

B représente un radical méthylène ou éthylène,

X a la signification déjà indiquée.

3. Dérivés répondant à la formule générale (I) de la revendication 1, ainsi que leurs sels, caractérisés en ce que dans ladite formule (I) ;

R et R′ représentent un atome d'hydrogène,

Y représente un radical méthyle, éthyle, n-propyle, isopropyle, n-butyle, tert-butyle, n-hexyle, cyclohexyle, allyle, phényle ou benzyle,

B représente un radical méthylène ou éthylène,

X représente un groupement amino, diméthylamino, diéthylamino, dipropylamino, diisopropylamino, dibutylamino, diisobutylamino, butylamino, cyclohexylamino, pipéridinoéthylamino, pyrrolidinyle, pipéridino, 2,3,4,5,6,7-hexahydroazépino, 3-azabicyclo [3,2,2] nonano, 2,6-diméthylpipéridino, 3,5-diméthylpipéridino, morpholino, pipérazin-1-yl, méthyl pipérazin-1-yl, hydroxyéthyl-pipérazin-1-yl, phénylpipérazin-1-yl, éthoxy carbonyle pipérazin-1-yl.

4. Dérivés répondant à la formule générale (I) de la revendication 1, ainsi que leurs sels, caractérisés en ce que dans ladite formule (I),

R et R′ représentent un atome d'hydrogène,

Y représente un radical méthyle, éthyle, n-propyle, isopropyle, n-butyle, tert-butyle, n-hexyle, cyclohexyle, allyle, phényle ou benzyle,

B représente un radical méthylène,

X représente un groupement pyrrolidinyle, pipéridino, 2, 3, 4, 5, 6, 7-hexahydroazépino, 3-azabicyclo [3,2,2] nonano, 2,6-diméthylpipéridino, 3,5-diméthylpipéridino.

5. La 1-pipéridinométhyl-4-éthyl [1,2,4] triazolo [4,3-a] quinazolin-5 (4H) one et ses sels.

6. La 1-pipéridinométhyl-4-n-propyl [1,2,4] triazolo [4,3-a] quinazolin-5 (4H) one et ses sels.

7. La 1-pipéridinométhyl-4-isopropyl [1,2,4] triazolo [4,3-a] quinazolin-5 (4H) one et ses sels.

8. La 1-pipéridinométhyl-4-n-butyl [1,2,4] triazolo [4,3-a] quinazolin-5 (4H) one et ses sels.

9. La 1-(2,3,4,5,6,7-hexahydrozépino) méthyl-4-n-butyl [1,2,4] triazolo [4,3-a] quinazolin-5 (4H) one et ses sels.

10. La 1-pipéridinométhyl-4-allyl [1,2,4] triazolo [4,3-a] quinazolin-5 (4H) one et ses sels.

11. Procédé de préparation des nouvelles triazoloquinazolones telles que définies à la revendication 1, ainsi que de leurs sels, caractérisé en ce que l'on fait réagir un produit de formule (II) :

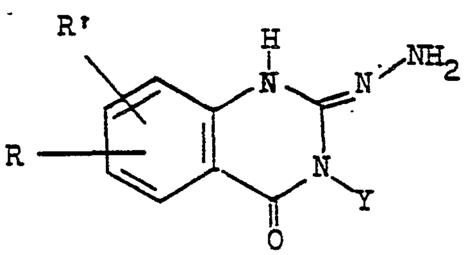

(II)

dans laquelle R, R′ et Y ont la signification déjà indiquée, avec le dérivé halogéné d'un halogénure d'acyle de formule (III) :

$$Hal—B—CO—Hal_1 \qquad (III)$$

dans laquelle Hal et $Hal_1$ représentent un atome de chlore ou de brome et B a la signification déjà indiquée, pour obtenir un produit de formule (IV) :

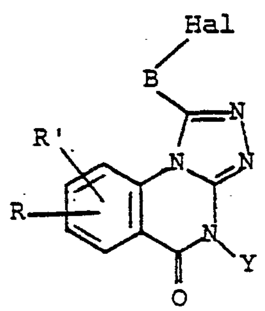

(IV)

dans laquelle R, R′, Y, B et Hal ont la signification déjà indiquée, le cas échéant, après avoir isolé puis cyclisé le produit intermédiaire de formule ($II_a$) :

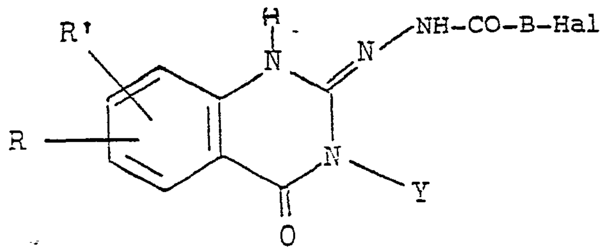

(IIa)

dans laquelle R, R', Y, B et Hal ont la signification déjà indiquée, puis fait réagir le produit de formule (IV) avec un produit de formule (V) :

H—X (V)

dans laquelle X a la signification déjà indiquée, pour obtenir le produit de formule (I) recherché que l'on peut, le cas échéant, salifier.

12. Procédé selon la revendication 11, caractérisé en ce que :

a) la réaction du produit de formule (II) avec le dérivé halogéné de l'halogénure d'acyle de formule (III) est effectuée au sein d'un solvant organique tel que le diméthylformamide et le cas échéant, en présence d'un agent alcalin ;

b) le dérivé halogéné de l'halogénure d'acyle de formule (III) est le dérivé chloré du chlorure d'acyle ;

c) la cyclisation du produit de formule ($II_a$) est effectuée par chauffage en présence d'un acide ;

d) la réaction du produit de formule (IV) avec le produit de formule (V) est effectuée au sein d'un solvant organique tel que le toluène et, le cas échéant, en présence d'un agent alcalin.

13. Procédé selon la revendication 11, pour la préparation des composés de formule (I), dans laquelle Y représente un radical alcoyle renfermant de 2 à 4 atomes de carbone, caractérisé en ce que l'on réduit les composés correspondant de formule (I), dans laquelle Y représente un radical alcényle renfermant de 2 à 4 atomes de carbone.

14. Médicaments caractérisés en ce qu'ils sont constitués par les nouvelles triazoloquinazolones telles que définies par la formule générale (I) de la revendication 1, ainsi que par leurs sels pharmaceutiquement acceptables.

15. Médicaments, caractérisés en ce qu'ils sont constitués par les nouvelles triazoloquinazolones, telles que définies à la revendication 2, 3 ou 4, ainsi que par leurs sels pharmaceutiquement acceptables.

16. Médicaments, caractérisés en ce qu'ils sont constitués par les nouvelles triazoloquinazolones telles que définies à l'une quelconque des revendications 5, 6, 7, 8, 9 ou 10, ainsi que par leurs sels pharmaceutiquement acceptables.

17. Compositions pharmaceutiques, caractérisées en ce qu'elles renferment, à titre de principe actif, l'un au moins des médicaments tels que définis à l'une quelconque des revendications 14, 15 ou 16.

**Revendications** (pour l'Etat contractant AT)

1. Procédé pour préparer les nouvelles triazoloquinazolones ainsi que leurs sels, répondant à la formule générale (I) :

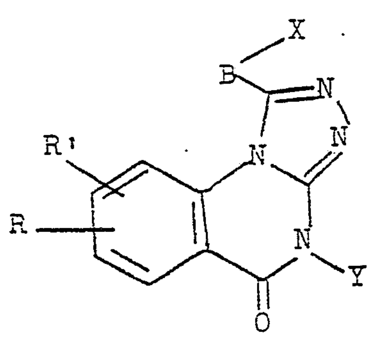

(I)

dans laquelle

R et R', identiques ou différents, représentent un atome d'hydrogène, un atome d'halogène, un radical alcoyle renfermant de 1 à 3 atomes de carbone, un radical alcoxy renfermant de 1 à 3 atomes de carbone, un radical nitro,

Y représente un radical alcoyle, linéaire ou ramifié, renfermant de 1 à 6 atomes de carbone, un radical cycloalcoyle renfermant de 3 à 8 atomes de carbone, un radical alcényle renfermant de 2 à 4 atomes de carbone, un radical aryle renfermant de 6 à 8 atomes de carbone, ou aralcoyle renfermant 7 ou 8 atomes de carbone,

B représente un groupement

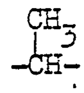

ou un radical alcoylène $-(CH_2)_n-$ dans lequel n représente un nombre entier égal à 1, 2 ou 3,

X représente un groupement

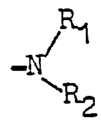

dans lequel $R_1$ et $R_2$, identiques ou différents, représentent un atome d'hydrogène, un groupement alcoyle, linéaire ou ramifié, renfermant de 1 à 6 atomes de carbone, un radical cycloalcoyle renfermant de 3 à 8 atomes de carbone, un radical aryle ou aralcoyle renfermant de 6 à 8 atomes de carbone, un radical aminoalcoyle, monoalcoyl ou dialcoylaminoalcoyle dans lesquels les groupements alcoyles renferment de 2 à 4 atomes de carbone, un radical pipéridinoalcoyle, morpholino-alcoyle ou pipérazinylalcoyle ou $R_1$ et $R_2$ forment ensemble avec l'atome d'azote auxquels ils sont liés un hétérocycle saturé mono ou bicyclique renfermant de 4 à 8 atomes de carbone pouvant être substitué par 1 ou 2 radicaux méthyle, ou pouvant renfermer en outre un atome d'oxygène, de soufre et d'azote, ledit atome d'azote pouvant être substitué par un radical alcoyle renfermant de 1 à 3 atomes de carbone, par un radical hydroxyalcoyle renfermant de 1 à 3 atomes de carbone, par un radical aryle ou aralcoyle renfermant de 6 à 8 atomes de carbone, ou par un radical alcoxycarbonyle renfermant de 2 à 5 atomes de carbone, ou enfin, X représente un radical

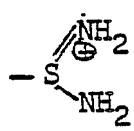 Cl ⊖ ou 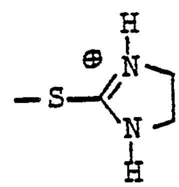 Cl ⊖

caractérisé en ce que l'on fait réagir un produit de formule (II) :

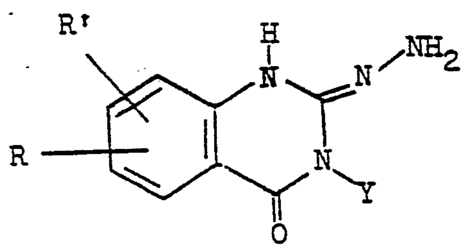

(II)

dans laquelle R, R' et Y ont la signification déjà indiquée, avec le dérivé halogéné d'un halogénure d'acyle de formule (III) :

$$Hal—B—CO—Hal_1 \quad (III)$$

dans laquelle Hal et $Hal_1$ représentent un atome de chlore ou de brome et B a la signification déjà indiquée, pour obtenir un produit de formule (IV) :

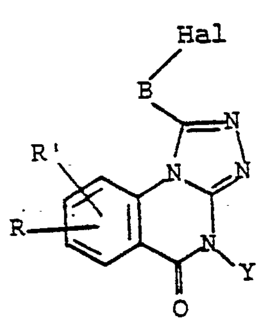

(IV)

dans laquelle R, R', Y, B et Hal ont la signification déjà indiquée, le cas échéant, après avoir isolé puis cyclisé le produit intermédiaire de formule ($II_a$) :

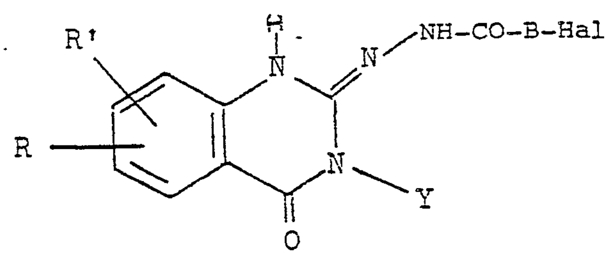

(IIa)

dans laquelle R, R', Y, B et Hal ont la signification déjà indiquée, puis fait réagir le produit de formule (IV) avec un produit de formule (V) :

H—X (V)

dans laquelle X a la signification déjà indiquée, pour obtenir le produit de formule (I) recherché que l'on peut, le cas échéant, salifier.

2. Procédé selon la revendication 1, caractérisé en ce que :

a) la réaction du produit de formule (II) avec le dérivé halogéné de l'halogénure d'acyle de formule (III) est effectuée au sein d'un solvant organique tel que le diméthylformamide et le cas échéant, en présence d'un agent alcalin ;

b) le dérivé halogéné de l'halogénure d'acyle de formule (III) est le dérivé chloré du chlorure d'acyle ;

c) la cyclisation du produit de formule ($II_a$) est effectuée par chauffage en présence d'un acide ;

d) la réaction du produit de formule (IV) avec le produit de formule (V) est effectuée au sein d'un solvant organique tel que le toluène et, le cas échéant, en présence d'un agent alcalin.

3. Procédé selon la revendication 1, pour la préparation des composés de formule (I), dans laquelle Y représente un radical alcoyle renfermant de 2 à 4 atomes de carbone, caractérisé en ce que l'on réduit les composés correspondant de formule (I), dans laquelle Y représente un radical alcényle renfermant de 2 à 4 atomes de carbone.

4. Procédé selon la revendication 1 ou 2, caractérisé en ce que l'on utilise au départ un composé de formule (II) dans laquelle

R et R', identiques ou différents, représentent un atome d'hydrogène, un atome de chlore, un radical méthyle, un radical méthoxy ou un radical nitro,

Y représente un radical alcoyle, linéaire ou ramifié, renfermant de 1 à 6 atomes de carbone, un radical cyclohexyle, un radical allyle, un radical phényle ou benzyle et un composé de formule (III) dans laquelle B représente un radical méthylène ou éthylène.

5. Procédé selon la revendication 1 ou 2, caractérisé en ce que l'on utilise au départ un composé de formule (II) dans laquelle

R et R' représentent un atome d'hydrogène,

Y représente un radical méthyle, éthyle, n-propyle, isopropyle, n-butyle, tert-butyle, n-hexyle, cyclohexyle, allyle, phényle ou benzyle, un composé de formule (III) dans laquelle B représente un radical méthylène ou éthylène et un composé de formule (V) dans laquelle X représente un groupement amino, diméthylamino, diéthylamino, dipropylamino, diisopropylamino, dibutylamino, diisobutylamino, butylamino, cyclohexylamino, pipéridinoéthylamino, pyrrolidinyle, pipéridino, 2,3,4,5,6,7-hexahydroazépino, 3-azabicyclo [3,2,2] nonano, 2,6-dimethylpipéridino, 3,5-diméthylpipéridino, morpholino, pipérazin-1-yl, méthyl pipérazin-1-yl, hydroxyéthyl pipérazin-1-yl, phénylpipérazin-1-yl, éthoxycarbonyle pipérazin-1-yl.

6. Procédé selon la revendication 1 ou 2, caractérisé en ce que l'on utilise au départ un composé de formule (II) dans laquelle

R et R' représentent un atome d'hydrogène,

Y représente un radical méthyle, éthyle, n-propyle, isopropyle, n-butyle, tert-butyle, n-hexyle, cyclohexyle, allyle, phényle ou benzyle, un composé de formule (III) dans laquelle B représente un radical méthylène et un composé de formule (V) dans laquelle X représente un groupement pyrrolidinyle, pipéridino, 2,3,4,5,6,7-hexahydroazépino, 3-azabicyclo [3,2,2] nonano, 2,6-diméthylpipéridino, 3,5-diméthylpipéridino.

7. Procédé selon la revendication 1 ou 2, caractérisé en ce que l'on prépare l'un quelconque des composés de formule (I) dont les noms suivent :

la 1-pipéridinométhyl-4-éthyl [1,2,4] triazolo [4,3-a] quinazolin-5 (4H) one et ses sels ;

la 1-pipéridinométhyl-4-n-propyl [1,2,4] triazolo [4,3-a] quinazolin-5 (4H) one et ses sels ;

la 1-pipéridinométhyl-4-isopropyl [1,2,4] triazolo [4,3-a] quinazolin-5 (4H) one et ses sels ;

la 1-pipéridinométhyl-4-n-butyl [1,2,4] triazolo [4,3-a] quinazolin-5 (4H) one et ses sels ;

la 1-(2,3,4,5,6,7-hexahydroazépino) méthyl-4-n-butyl [1,2,4] triazolo [4,3-a] quinazolin-5 (4H) one et ses sels ;

la 1-pipéridinométhyl-4-allyl [1,2,4] triazolo [4,3-a] quinazolin-5 (4H) one et ses sels.

**Claims** (for the Contracting States : BE, CH, DE, FR, IT, LI, LU, NL, SE)

1. New triazoloquinazolones, as well as their salts, characterized in that they answer to the general formula (I) :

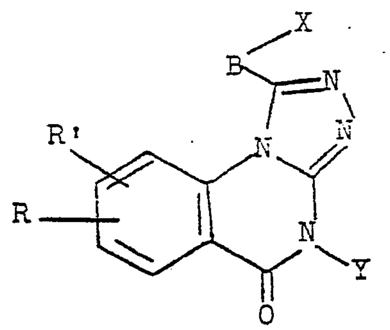

(I)

in which

R and R', identical or different, represent a hydrogen atom, a halogen atom, an alkyl radical containing from 1 to 3 carbon atoms, an alcoxy radical containing from 1 to 3 carbon atoms, or a nitro radical,

Y represents an alkyl radical, linear or branched, containing from 1 to 6 carbon atoms, a cycloalkyl radical containing from 3 to 8 carbon atoms, an alkenyl radical containing from 2 to 4 carbon atoms, an aryl radical containing from 6 to 8 carbon atoms, or an aralkyl containing 7 or 8 carbon atoms,

B represents a

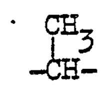

group or an alkylene radical $-(CH_2)n-$ in which n represents a whole number 1, 2 or 3,

X represents a

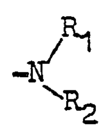

in which $R_1$ and $R_2$, identical or different, each represent a hydrogen atom, an alkyl group, linear or branched, containing from 1 to 6 carbon atoms, a cycloalkyl radical containing from 3 to 8 carbon atoms, an aryl or aralkyl radical containing from 6 to 8 carbon atoms, an aminoalkyl, monoalkyl- or dialkylaminoalkyl radical in which the alkyl groups contain from 2 to 4 carbon atoms, a piperidino-alkyl, morpholinoalkyl or piperazinylalkyl radical or $R_1$ and $R_2$ form together with the nitrogen atom to which they are linked a mono or bicyclic saturated heterocycle containing from 4 to 8 carbon atoms able to be substituted by 1 or 2 methyl radicals, or able to contain, in addition, an oxygen, sulphur and nitrogen atom, the said nitrogen atom able to be substituted by an alkyl radical containing from 1 to 3 carbon atoms, by a hydroxyalkyl radical containing from 1 to 3 carbon atoms, by an aryl or aralkyl radical containing from 6 to 8 carbon atoms, or by an alcoxy-carbonyl radical containing from 2 to 5 carbon atoms, or finally, X represents a radical

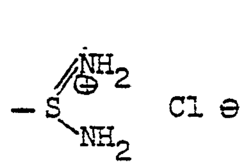

or

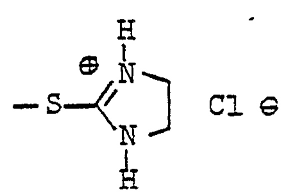

2. Derivatives answering to the general formula (I) of claim 1, as well as their salts, characterized in that in the said formula (I),

R and R', identical or different, represent a hydrogen atom, a chlorine atom, a methyl radical, a methoxy radical or a nitro radical,

Y represents an alkyl radical, linear or branched, containing from 1 to 6 carbon atoms, a cyclohexyl radical, an allyl radical, a phenyl or benzyl radical,

B represents a methylene or ethylene radical.

X has the significance already indicated.

3. Derivatives answering to the general formula (I) of claim 1, as well as their salts, characterized in that in the said formula (I),

R and R' represent a hydrogen atom,

Y represents a methyl, ethyl, n-propyl, isopropyl, n-butyl, tert-butyl, n-hexyl, cyclohexyl, allyl, phenyl or benzyl radical,

B represents a methylene or ethylene radical,

X represents one of the following groups : an amino, dimethylamino, diethylamino, dipropylamino, diisopropylamino, dibutylamino, diisobutylamino, butylamino, cyclohexylamino, piperidinoethylamino, pyrrolidinyl, piperidino, 2,3,4,5,6,7-hexahydroazepino, 3-azabicyclo-[3,2,2] nonano, 2,6-dimethyl-piperidino, 3,5-dimethyl-piperidino, morpholino, piperazin-1-yl, methyl piperazin-1-yl, hydroxyethyl-piperazin-1-yl, phenylpiperazin-1-yl, or ethoxy carbonyl piperazin-1-yl.

4. Derivatives answering to the general formula (I) of claim 1, as well as their salts, characterized in that in the said formula (I),

R and R' represent a hydrogen atom,

Y represents a methyl, ethyl, n-propyl, isopropyl, n-butyl, tertbutyl, n-hexyl, cyclo-hexyl, allyl, phenyl or benzyl radical,

B represents a methylene radical,

X represents a pyrrolidinyl, piperidino, 2,3,4,5,6,7-hexahydroazepino, 3-azabicyclo-[3,2,2] nonano, 2,6-dimethylpiperidino, or 3,5-dimethylpiperidino group.

5. 1-piperidinomethyl-4-ethyl [1,2,4] triazolo [4,3-a] quinazolin-5-(4H)-one and its salts.

6. 1-piperidinomethyl-4-n-propyl [1,2,4] triazolo [4,3-a] quinazolin-5-(4H)-one and its salts.

7. 1-piperadinomethyl-4-isopropyl [1,2,4] triazolo [4,3-a] quinazolin-5-(4H)-one and its salts.

8. 1-piperidinomethyl-4-n-butyl [1,2,4] triazolo [4,3-a] quinazolin-5-(4H)-one and its salts.

9. 1-(2,3,4,5,6,7-hexahydroazepino)methyl-4-n-butyl [1,2,4] triazolo [4,3-a] quinazolin-5-(4H)-one and its salts.

10. 1-piperidinomethyl-4-allyl [1,2,4] triazolo [4,3-a] quinazolin-5-(4H)-one and its salts.

11. Preparation process of new triazoloquinazolones as defined in claim 1, as well as their salts, characterized in that a product with the formula (II) :

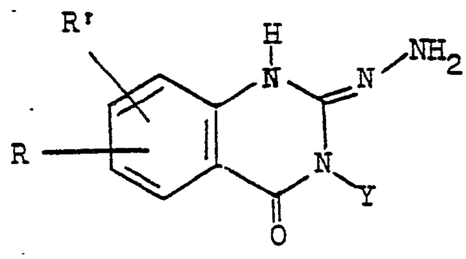

(II)

in which R, R' and Y have the significance already indicated, is made to react with the halogenated derivative of an acyl halogenide with the formula (III) :

$$Hal—B—CO—Hal_1 \qquad (III)$$

in which Hal and $Hal_1$ represent a chlorine or bromine atom and B has the significance already indicated, so as to obtain a product with the formula (IV) :

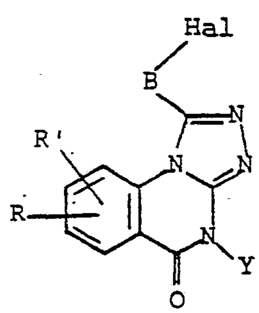

(IV)

in which R, R', Y, B and Hal have the significance already indicated, if necessary, after having isolated then cyclized the intermediate product with the formula ($II_a$) :

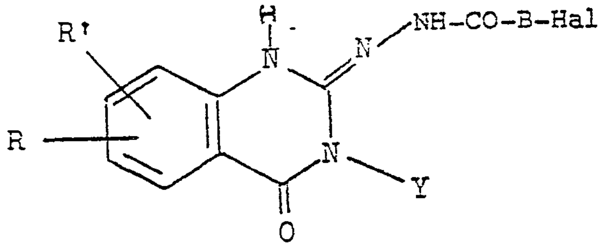

(IIa)

in which R, R', Y, B and Hal have the significance already indicated, then the product with the formula (IV) is made to react with a product with the formula (V) :

$$H—X \quad (V)$$

in which X has the significance already indicated, so as to obtain the product sought with the formula (I) which can, if necessary, be salified.

12. Process according to claim 11, characterized in that :

a) the reaction of the product with the formula (II) with the halogenated derivative of the acyl halogenide with the formula (III) is carried out in an organic solvent such as dimethylformamide and if necessary, in the presence of an alkaline agent ;

b) the halogenated derivative of the acyl halogenide with the formula (III) is the chlorated derivative of acyl chloride ;

c) the cyclization of the product with the formula ($II_a$) is carried out by heating in the presence of an acid ;

d) the reaction of the product with the formula (IV) with the product with the formula (V) is carried out in an organic solvent such as toluene and, if necessary, in the presence of an alkaline agent.

13. Process according to claim 11, for the preparation of compounds with the formula (I), in which Y represents an alkyl radical containing from 2 to 4 carbon atoms, characterized in that the corresponding compounds with the formula (I) in which Y represents an alkenyl radical containing from 2 to 4 carbon atoms, are reduced.

14. Medicaments, characterized in that they are constituted by new triazoloquinazolones as defined by the general formula (I) of claim 1, as well as by their pharmaceutically acceptable salts.

15. Medicaments, characterized in that they are constituted by new triazoloquinazolones, as defined in claim 2, 3, or 4, as well as by their pharmaceutical acceptable salts.

16. Medicaments, characterized in that they are constituted by new triazoloquinazolones as defined in any one of the claims 5, 6, 7, 8, 9 or 10, as well as by their pharmaceutically acceptable salts.

17. Pharmaceutical compositions, characterized in that they contain, as active principle, one at least of the medicaments as defined in any one of the claims 14, 15 or 16.

**Claims** (for the Contracting State AT)

1. Process for preparing new triazoloquinazolones as well as their salts, answering to the general formula (I) :

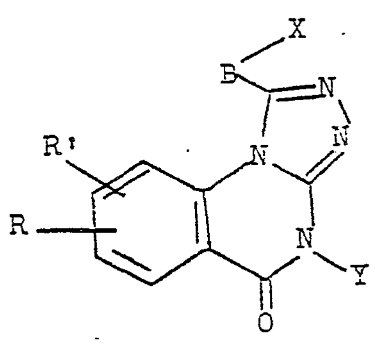

(I)

in which

R and R', identical or different, represent a hydrogen atom, a halogen atom, an alkyl radical containing from 1 to 3 carbon atoms, an alcoxy radical containing from 1 to 3 carbon atoms, or a nitro radical,

Y represents an alkyl radical, linear or branched, containing from 1 to 6 carbon atoms, a cycloalkyl radical containing from 3 to 8 carbon atoms, an alkenyl radical containing from 2 to 4 carbon atoms, an aryl radical containing from 6 to 8 carbon atoms or an aralkyl radical containing 7 or 8 carbon atoms,

B represents a

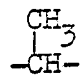

group or an $-(CH_2)n-$ alkylene radical in which n represents a whole number 1, 2 or 3,

X represents a

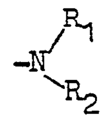

group in which $R_1$ and $R_2$, identical or different, each represent a hydrogen atom, an alkyl group, linear or branched, containing from 1 to 6 carbon atoms, a cycloalkyl radical containing from 3 to 8 carbon atoms, an aryl or aralkyl radical containing from 6 to 8 carbon atoms, an aminoalkyl, monoalkyl- or dialkylaminoalkyl radical in which the alkyl groups contain from 2 to 4 carbon atoms, a piperidinoalkyl, morpholinoalkyl or piperazinylalkyl radical or $R_1$ and $R_2$ form, together with the nitrogen atom to which they are linked, a mono- or bicyclic saturated heterocyle containing from 4 to 8 carbon atoms able to be substituted by 1 or 2 methyl radicals, or able to contain in addition an oxygen, sulphur and nitrogen atom, the said nitrogen atom able to be substituted by an alkyl radical containing from 1 to 3 carbon atoms, by a hydroxyalkyl radical containing from 1 to 3 carbon atoms, by an aryl or aralkyl radical containing from 6 to 8 carbon atoms, or by an alcoxycarbonyl radical containing from 2 to 5 carbon atoms, or finally, X represents a radical

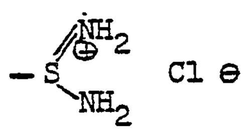

or

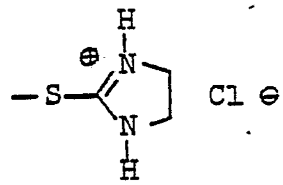

characterized in that a product with the formula (II) :

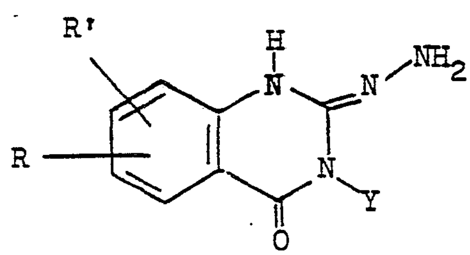

(II)

in which R, R′ and Y have the significance already indicated, is made to react with a halogenated derivative of an acyl halogenide with the formula (III) :

$$Hal-B-CO-Hal_1 \qquad (III)$$

in which Hal and $Hal_1$ represent a chlorine or bromine atom and B has the significance already indicated, so as to obtain a product with the formula (IV) :

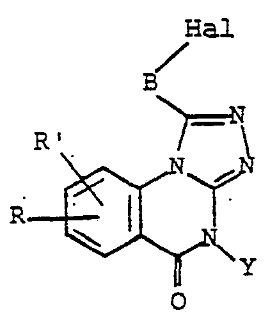

(IV)

in which R, R', Y, B and Hal have the significance already indicated, if necessary, after having isolated then cyclized the intermediate product with the formula ($II_a$) :

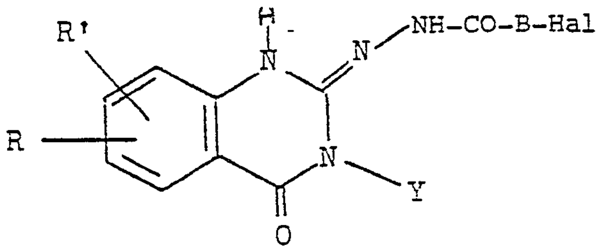

(IIa)

in which R, R', Y, B and Hal have the significance already indicated, then the product with the formula (IV) is made to react with a product with the formula (IV) :

$$H—X \qquad (V)$$

in which X has the significance already indicated, so as to obtain the product sought with the formula (I), which can, if necessary, be salified.

2. Process according to claim 1, characterized in that :

a) the reaction of the product with the formula (II) with the halogenated derivative of the acyl halogenide with the formula (III) is carried out in an organic solvent such as dimethylformamide and if necessary, in the presence of an alkaline agent ;

b) the halogenated derivative of the acyl halogenide with the formula (III) is the chlorated derivative of the acyl chloride ;

c) the cyclization of the product with the formula ($II_a$) is carried out by heating in the presence of an acid ;

d) the reaction of the product with the formula (IV) with the product with the formula (V) is carried out in an organic solvent such as toluene and, if necessary, in the presence of an alkaline agent.

3. Process according to claim 1, for the preparation of compounds with the formula (I), in which Y represents an alkyl radical containing from 2 to 4 carbon atoms, characterized in that the corresponding compounds with the formula (1), in which Y represents an alkenyl radical containing from 2 to 4 carbon atoms, are reduced.

4. Process according to claim 1 or 2, characterized in that at the start a compound with the formula (II) is used, in which

R and R', identical or different, represent a hydrogen atom, a chlorine atom, a methyl radical, a methoxy radical or a nitro radical,

Y represents an alkyl radical, linear or branched, containing from 1 to 6 carbon atoms, a cyclohexyl radical, an allyl radical, a phenyl or benzyl radical and a compound with the formula (III) in which B represents a methylene or ethylene radical.

5. Process according to claim 1 or 2, characterized in that at the start a compound with the formula (II) is used in which

R and R' represent a hydrogen atom,

Y represents a methyl, ethyl, n-propyl, isopropyl, n-butyl, tert-butyl, n-hexyl, cyclohexyl, allyl, phenyl or benzyl radical, a compound with the formula (III) in which B represents a methylene or ethylene radical and a compound with the formula (V) in which X represents one of the following groups : an amino, dimethylamino, diethylamino, dipropylamino, diisopropylamino, dibutylamino, diisobutylamino, butylamino cyclohexylamino, piperidinoethylamino, pyrrolidinyl, piperidino, 2,3,4,5,6,7-hexahydroazepino, 3-azabicyclo-[3,2,2] nonano, 2,6-dimethylpiperidino, 3,5-dimethylpiperidino, morpholino, piperazin-1-yl, methyl piperazin-1-yl, hydroxyethyl piperazin-1-yl, phenyl- piperazin-1-yl, or ethoxycarbonyl piperazin-1-yl.

6. Process according to claim 1 or 2, characterized in that at the start a compound with the formula (II) is used in which

R and R' represent a hydrogen atom,

Y represents a methyl, ethyl, n-propyl, isopropyl, n-butyl, tert-butyl, n-hexyl, cyclohexyl, allyl, phenyl or benzyl radical, a compound with the formula (III) in which B represents a methylene radical and a compound with the formula (V) in which X represents a pyrrolidinyl, piperidino, 2,3,4,5,6,7-hexahydroazepino, 3-azabicyclo-[3,2,2] nonano, 2,6-dimethylpiperidino, or 3,5-dimethylpiperidino group.

7. Process according to claim 1 or 2, characterized in that any one of the compounds with the formula (I) are prepared, of which the names follow :

1-piperidinomethyl-4-ethyl-[1,2,4] triazolo-[4,3-a] quinazolin-5-(4H)-one and its salts ;

1-piperidinomethyl-4-n-propyl-[1,2,4] triazolo-[4,3-a]-quinazolin-5-(4H)-one and its salts ;

1-piperidinomethyl-4-isopropyl-[1,2,4] triazolo-[4,3-a]-quinazolin-5-(4H)-one and its salts ;

1-piperidinomethyl-4-n-butyl-[1,2,4] triazolo-[4,3-a]-quinazolin-5-(4H)-one and its salts ;

1-(2,3,4,5,6,7-hexahydroazepino)methyl-4-n-butyl-[1,2,4]-triazolo-[4,3-a] quinazolin-5-(4H)-one and its salts ;

1-piperidinomethyl-4-allyl-[1,2,4] triazolo-[4,3-a]-quinazolin-5-(4H)-one and its salts.

**Patentansprüche** (für die Vertragsstaaten : BE, CH, DE, FR, IT, LI, LU, NL, SE)

1. Neue Triazolochinazolone sowie deren Salze, dadurch gekennzeichnet, daß sie der allgemeinen Formel (I)

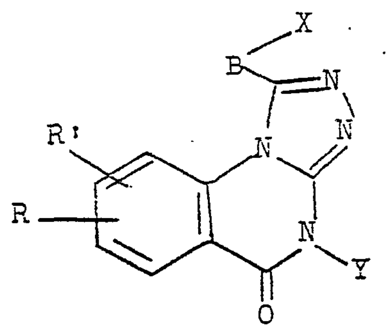

(I)

entsprechen, worin

R und R', die gleich oder verschieden sind, ein Wasserstoffatom, ein Halogenatom, einen Alkylrest mit 1 bis 3 Kohlenstoffatomen, einen Alkoxyrest mit 1 bis 3 Kohlenstoffatomen, eine Nitrogruppe bedeuten,

Y einen linearen oder verzweigten Alkylrest mit 1 bis 6 Kohlenstoffatomen, einen Cycloalkylrest mit 3 bis 8 Kohlenstoffatomen, einen Alkenylrest mit 2 bis 4 Kohlenstoffatomen, einen Arylrest mit 6 bis 8 Kohlenstoffatomen oder einen Aralkylrest mit 7 oder 8 Kohlenstoffatomen bedeutet,

B eine Gruppe

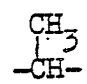

oder einen Alkylenrest $-(CH_2)_n$ darstellt, worin n eine ganze Zahl entsprechend 1, 2 oder 3 bedeutet,

X eine Gruppe

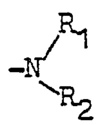

bedeutet, worin $R_1$ und $R_2$, die gleich oder verschieden sind, ein Wasserstoffatom, eine lineare oder verzweigte Alkylgruppe mit 1 bis 6 Kohlenstoffatomen, einen Cycloalkylrest mit 3 bis 8 Kohlenstoffatomen, einen Aryl- oder Aralkylrest mit 6 bis 8 Kohlenstoffatomen, einen Aminoalkyl-, Monoalkyl- oder Dialkylaminoalkylrest, worin die Alkylgruppen 2 bis 4 Kohlenstoffatome enthalten, einen Piperidinoalkyl-, Morpholinoalkyl- oder Piperazinylalkyl-Rest darstellen oder $R_1$ und $R_2$ gemeinsam mit dem Stickstoffatom, an das sie gebunden sind, einen gesättigten mono- oder bicyclischen Heterocyclus mit 4 bis 8 Kohlenstoffatomen bilden, der durch einen oder zwei Methylreste substituiert sein kann oder außerdem ein Sauerstoff-, Schwefel- oder Stickstoffatom enthalten kann, wobei dieses Stickstoffatom durch einen Alkylrest mit 1 bis 3 Kohlenstoffatomen, durch einen Hydroxyalkylrest mit 1 bis 3 Kohlenstoffatomen, durch einen Aryl- oder Aralkylrest mit 6 bis 8 Kohlenstoffatomen oder durch einen Alkoxycarbonylrest mit 2 bis 5 Kohlenstoffatomen substituiert sein kann, oder schließlich X einen Rest

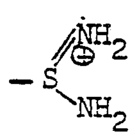 Cl ⊖ oder 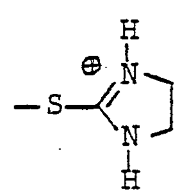 Cl ⊖

bedeutet.

2. Derivate der allgemeinen Formel (I) gemäß Anspruch 1 sowie deren Salze, dadurch gekennzeichnet, daß in der Formel (I)

R und R', die gleich oder verschieden sind, ein Wasserstoffatom, ein Chloratom, eine Methylgruppe, eine Methoxygruppe, oder eine Nitrogruppe bedeuten,

Y einen linearen oder verzweigten Alkylrest mit 1 bis 6 Kohlenstoffatomen, einen Cyclohexylrest, einen Allylrest, einen Phenyl- oder Benzylrest bedeutet,

B eine Methylen- oder Ethylengruppe darstellt,

X die angegebene Bedeutung besitzt.

3. Derivate der allgemeinen Formel (I) gemäß Anspruch 1 sowie deren Salze, dadurch gekennzeichnet, daß in der Formel (I)

R und R' ein Wasserstoffatom bedeuten,

Y einen Methyl-, Ethyl-, n-Propyl-, Isopropyl-, n-Butyl-, tert.-Butyl-, n-Hexyl-, Cyclohexyl-, Allyl-, Phenyl- oder Benzylrest bedeutet,

B eine Methylen- oder Ethylengruppe darstellt,

X eine Amino-, Dimethylamino-, Diethylamino-, Dipropylamino-, Diisopropylamino-, Dibutylamino-, Diisobutylamino-, Butylamino-, Cyclohexylamino-, Piperidinoethylamino-, Pyrrolidinyl- Piperidino-, 2,3,4,5,6,7-Hexahydroazepino-, 3-Aza-bicyclo [3,2,2] nonano-, 2,6-Dimethylpiperidino-, 3,5-Dimethylpiperidino-, Morpholino-, Piperazin-1-yl-, Methylpiperazin-1-yl-, Hydroxyethylpiperazin-1-yl-, Phenylpiperazin-1-yl- oder Ethoxycarbonyl-piperazin-1-yl-Gruppe bedeutet.

4. Derivate der allgemeinen Formel (I) gemäß Anspruch 1 sowie deren Salze, dadurch gekennzeichnet, daß in der Formel (I)

R und R' ein Wasserstoffatom bedeuten,

Y einen Methyl-, Ethyl-, n-Propyl-, Isopropyl-, n-Butyl-, tert.-Butyl-, n-Hexyl-, Cyclohexyl-, Allyl-, Phenyl- oder Benzylrest darstellt,

B eine Methylengruppe bedeutet,

X eine Pyrrolidinyl-, Piperidino-, 2,3,4,5,6,7-Hexahydroazepino-, 3-Aza-bicyclo [3,2,2] nonano-, 2,6-Dimethylpiperidino- oder 3,5-Dimethylpiperidino-Gruppe bedeutet.

5. 1-Piperidinomethyl-4-ethyl [1,2,4] triazolo [4,3-a]-chinazolin-5-(4H)-on und dessen Salze.

6. 1-Piperidinomethyl-4-n-propyl [1,2,4] triazolo [4,3-a] chinazolin-5-(4H)-on und dessen Salze.

7. 1-Piperidinomethyl-4-isopropyl [1,2,4] triazolo-[4,3-a] chinazolin-5-(4H)-on und dessen Salze.

8. 1-Piperidinomethyl-4-n-butyl [1,2,4] triazolo [4,3-a] chinazolin-5-(4H)-on und dessen Salze.

9. 1-(2,3,4,5,6,7-Hexahydroazepino)-methyl-4-n-butyl [1,2,4] triazolo [4,3-a] chinazolin-5-(4H)-on und dessen Salze.

10. 1-Piperidinomethyl-4-allyl [1,2,4] triazolo [4,3-a]-chinazolin-5-(4H)-on und dessen Salze.

11. Verfahren zur Herstellung neuer Triazolochinazolone gemäß Anspruch 1 sowie von deren Salzen, dadurch gekennzeichnet, daß man ein Produkt der Formel (II)

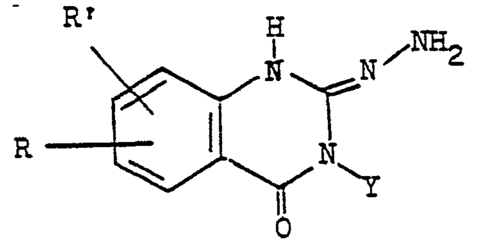

(II)

worin R, R' und Y die angegebene Bedeutung besitzen, mit einem Halogenderivat eines Acylhalogenids der Formel (III)

$$Hal—B—CO—Hal_1 \quad (III)$$

umsetzt, worin Hal und $Hal_1$ ein Chlor- oder Bromatom bedeuten und B die angegebene Bedeutung besitzt, um ein Produkt der Formel (IV)

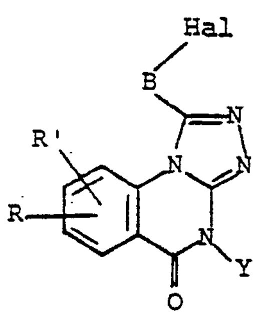

(IV)

worin R, R', Y, B und Hal die angegebene Bedeutung besitzen, gegebenenfalls nach Isolierung und anschließender Cyclisierung des Zwischenprodukts der Formel ($II_a$)

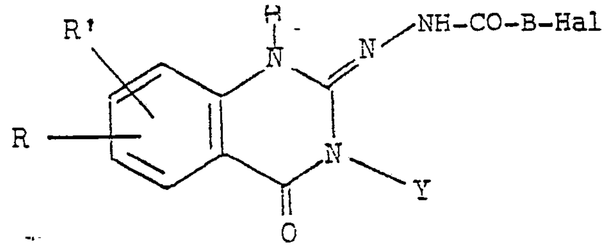

(IIa)

worin R, R', Y, B und Hal die angebene Bedeutung besitzen, zu erhalten, und dann das Produkt der Formel (IV) mit einem Produkt der Formel (V)

H—X (V)

umsetzt, worin X die angegebene Bedeutung besitzt, um das gewünschte Produkt der Formel (I) zu erhalten, das man gegebenenfalls in ein Salz überführen kann.

12. Verfahren gemäß Anspruch 11, dadurch gekennzeichnet, daß

a) die Umsetzung des Produkts der Formel (II) mit dem Halogenderivat des Acylhalogenids der Formel (III) in dem Medium eines organischen Lösungsmittels, wie Dimethylformamid, und gegebenenfalls in Anwesenheit eines alkalischen Mittels durchgeführt wird;

b) das Halogenderivat des Acylhalogenids der Formel (III) das Chlorderivat eines Acylchlorids ist;

c) die Cyclisierung des Produkts der Formel ($II_a$) durch Erhitzen in Gegenwart einer Säure erfolgt;

d) die Umsetzung des Produkts der Formel (IV) mit dem Produkt der Formel (V) in dem Medium eines organischen Lösungsmittels, wie Toluol, und gegebenenfalls in Anwesenheit eines alkalischen Mittels erfolgt.

13. Verfahren gemäß Anspruch 11 zur Herstellung von Verbindungen der Formel (I), worin Y einen Alkylrest mit 2 bis 4 Kohlenstoffatomen bedeutet, dadurch gekennzeichnet, daß man die entsprechenden Verbindungen der Formel (I) reduziert, worin Y einen Alkenylrest mit 2 bis 4 Kohlenstoffatomen bedeutet.

14. Arzneimittel, dadurch gekennzeichnet, daß sie aus den neuen Triazolochinazolonen der allgemeinen Formel (I) gemäß Anspruch 1 sowie aus deren pharmazeutisch verträglichen Salzen bestehen.

15. Arzneimittel, dadurch gekennzeichnet, daß sie aus den neuen Triazolochinazolonen gemäß Anspruch 2, 3 oder 4 sowie aus deren pharmazeutisch verträglichen Salzen bestehen.

16. Arzneimittel, dadurch gekennzeichnet, daß sie aus den neuen Triazolochinazolonen gemäß einem der Ansprüche 5, 6, 7, 8, 9 oder 10 sowie aus deren pharmazeutisch verträglichen Salzen bestehen.

17. Pharmazeutische Zusammensetzungen, dadurch gekennzeichnet, daß sie als Wirkstoff zumindest eines der Arzneimittel gemäß einem der Ansprüche 14, 15 oder 16 enthalten.

**Patentansprüche** (für den Vertragsstaat AT)

1. Verfahren zur Herstellung von neuen Triazolochinazolonen sowie von deren Salzen der allgemeinen Formel (I)

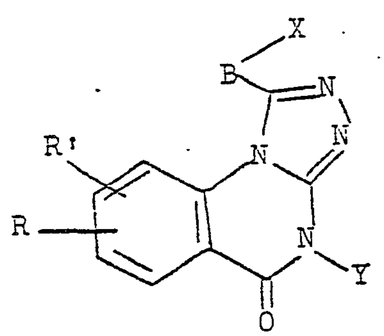

(I)

worin

R und R', die gleich oder verschieden sind, ein Wasserstoffatom, ein Halogenatom, einen Alkylrest mit 1 bis 3 Kohlenstoffatomen, einen Alkoxyrest mit 1 bis 3 Kohlenstoffatomen, eine Nitrogruppe bedeuten,

Y einen linearen oder verzweigten Alkylrest mit 1 bis 6 Kohlenstoffatomen, einen Cycloalkylrest mit 3 bis 8 Kohlenstoffatomen, einen Alkenylrest mit 2 bis 4 Kohlenstoffatomen, einen Arylrest mit 6 bis 8 Kohlenstoffatomen oder einen Aralkylrest mit 7 oder 8 Kohlenstoffatomen bedeutet,

B eine Gruppe

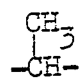

oder einen Alkylenrest $-(CH_2)_n$ darstellt, worin n eine ganze Zahl entsprechend 1, 2 oder 3 bedeutet,

X eine Gruppe

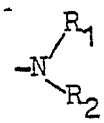

bedeutet, worin $R_1$ und $R_2$, die gleich oder verschieden sind, ein Wasserstoffatom, eine lineare oder verzweigte Alkylgruppe mit 1 bis 6 Kohlenstoffatomen, einen Cycloalkylrest mit 3 bis 8 Kohlenstoffatomen, einen Aryl- oder Aralkylrest mit 6 bis 8 Kohlenstoffatomen, einen Aminoalkyl-, Monoalkyl- oder Dialkylaminoalkylrest, worin die Alkylgruppen 2 bis 4 Kohlenstoffatome enthalten, einen Piperidinoalkyl, Morpholinoalkyl- oder Piperazinylalkyl-Rest darstellen oder $R_1$ und $R_2$ gemeinsam mit dem Stickstoffatom, an das sie gebunden sind, einen gesättigten mono- oder bicyclischen Heterocyclus mit 4 bis 8 Kohlenstoffatomen bilden, der durch einen oder zwei Methylreste substituiert sein kann oder außerdem ein Sauerstoff-, Schwefel- oder Stickstoffatom enthalten kann, wobei dieses Stickstoffatom durch einen Alkylrest mit 1 bis 3 Kohlenstoffatomen, durch einen Hydroxyalkylrest mit 1 bis 3 Kohlenstoffatomen, durch einen Aryl- oder Aralkylrest mit 6 bis 8 Kohlenstoffatomen oder durch einen Alkoxycarbonylrest mit 2 bis 5 Kohlenstoffatomen substituiert sein kann, oder schließlich X einen Rest

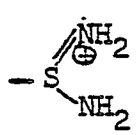 Cl ⊖ oder 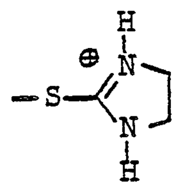 Cl ⊖

bedeutet, dadurch gekennzeichnet, daß man ein Produkt der Formel (II)

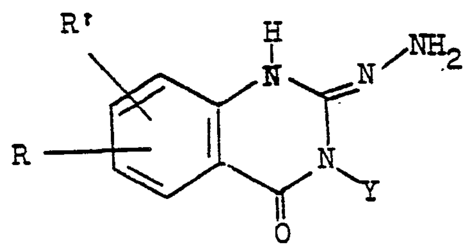

(II)

worin R, R′ und Y die angegebene Bedeutung haben, mit dem Halogenderivat eines Acylhalogenids der Formel (III)

$$Hal{-}B{-}CO{-}Hal_1 \qquad (III)$$

umsetzt, worin Hal und $Hal_1$ ein Chlor- oder Bromatom bedeuten und B die angegebene Bedeutung besitzt, um ein Produkt der Formel (IV)

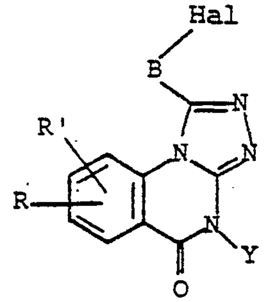

(IV)

worin R, R', Y, B und Hal die angegebene Bedeutung besitzen, gegebenenfalls nach Isolierung und anschließender Cyclisierung des Zwischenprodukts der Formel ($II_a$)

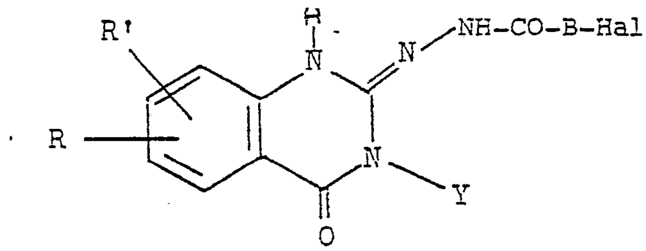

(IIa)

worin R, R', Y, B und Hal die angegebene Bedeutung besitzen, zu erhalten, und danach das Produkt der Formel (IV) mit einem Produkt der Formel (V)

H—X (V)

umsetzt, worin X die angegebene Bedeutung besitzt, um das gewünschte Produkt der Formel (I) zu erhalten, das man gegebenenfalls in ein Salz überführen kann.

2. Verfahren gemäß Anspruch 1, dadurch gekennzeichnet, daß

a) die Umsetzung des Produkts der Formel (II) mit dem Halogenderivat des Acylhalogenids der Formel (III) in dem Medium eines organischen Lösungsmittels, wie Dimethylformamid, und gegebenenfalls in Anwesenheit eines alkalischen Mittels durchgeführt wird ;

b) Das Halogenderivat des Acylhalogenids der Formel (III) das Chlorderivat eines Acylchlorids ist ;

c) die Cyclisierung des Produkts der Formel ($II_a$) durch Erhitzen in Gegenwart einer Säure durchgeführt wird ;

d) die Umsetzung des Produkts der Formel (IV) mit dem Produkt der Formel (V) in dem Medium eines organischen Lösungsmittels, wie Toluol, und gegebenenfalls in Anwesenheit eines alkalischen Mittels durchgeführt wird.

3. Verfahren gemäß Anspruch 1 zur Herstellung von Verbindungen der Formel (I), worin Y einen Alkylrest mit 2 bis 4 Kohlenstoffatomen bedeutet, dadurch gekennzeichnet, daß man die entsprechenden Verbindungen der Formel (I), worin Y einen Alkenylrest mit 2 bis 4 Kohlenstoffatomen darstellt, reduziert.

4. Verfahren gemäß Anspruch 1 oder 2, dadurch gekennzeichnet, daß man von einer Verbindung der Formel (II), worin R und R', die gleich oder verschieden sind, ein Wasserstoffatom, ein Chloratom, einen Methylrest, einen Methoxyrest oder eine Nitrogruppe bedeuten, Y einen linearen oder verzweigten Alkylrest mit 1 bis 6 Kohlenstoffatomen, einen Cyclohexylrest, einen Allylrest, einen Phenyl- oder Benzylrest bedeutet, und einer Verbindung der Formel (III), worin B eine Methylen- oder Ethylengruppe bedeutet, ausgeht.

5. Verfahren gemäß Anspruch 1 oder 2, dadurch gekennzeichnet, daß man von einer Verbindung der Formel (II), worin R und R' ein Wasserstoffatom bedeuten, Y einen Methyl-, Ethyl-, n-Propyl-, Isopropyl-, n-Butyl-, tert.-Butyl-, n-Hexyl-, Cyclohexyl-, Allyl-, Phenyl-, oder Benzylrest bedeutet, einer Verbindung der Formel (III), worin B eine Methylen- oder Ethylengruppe bedeutet, und einer Verbindung der Formel (V), worin X eine Amino-, Dimethylamino-, Diethylamino-, Dipropylamino-, Diisopropylamino-, Dibutylamino-, Diisobutylamino-, Butylamino-, Cyclohexylamino-, Piperidinoethylamino-, Pyrrolidinyl-, Piperidino-, 2,3,4,5,6,7-Hexahydroazepino-, 3-Aza-bicyclo [3,2,2] nonano-, 2,6-Dimethylpiperidino-, 3,5-Dimethylpiperidino-, Morpholino-, Piperazin-1-yl-, Methylpiperazin-1-yl, Hydroxyethylpiperazin-1-yl-, Phenyl-piperazin-1-yl- oder Ethoxycarbonylpiperazin-1-ylgruppe bedeutet, ausgeht.

6. Verfahren gemäß Anspruch 1 oder 2, dadurch gekennzeichnet, daß man von einer Verbindung der Formel (II), worin R und R' ein Wasserstoffatom bedeuten, Y einen Methyl-, Ethyl-, n-Propyl-, Isopropyl-, n-Butyl-, tert.-Butyl-, n-Hexyl-, Cyclohexyl-, Allyl-, Phenyl- oder Benzylrest darstellt, einer Verbindung der Formel (III), worin B eine Methylengruppe bedeutet, und einer Verbindung der Formel (V), worin X eine Pyrrolidinyl-, Piperidino-, 2,3,4,5,6,7-Hexahydroazepino-, 3-Aza-bicyclo [3,2,2] nonano-, 2,6-Dimethylpiperidino- oder 3,5-Dimethylpiperidinogruppe darstellt, ausgeht.

7. Verfahren gemäß Anspruch 1 oder 2, dadurch gekennzeichnet, daß man eine der Verbindungen der Formel (I) mit den folgenden Bezeichnungen herstellt :

1-Piperidinomethyl-4-ethyl [1,2,4] triazolo [4,3-a]-chinazolin-5-(4H)-on und dessen Salze :

1-Piperidinomethyl-4-n-propyl [1,2,4] triazolo [4,3-a] chinazolin-5-(4H)-on und dessen Salze ;

1-Piperidinomethyl-4-isopropyl [1,2,4] triazolo-[4,3-a] chinazolin-5-(4H)-on und dessen Salze ;

1-Piperidinomethyl-4-n-butyl [1,2,4] triazolo [4,3-a] chinazolin-5-(4H)-on und dessen Salze ;

1-(2,3,4,5,6,7-Hexahydroazepino)-methyl-4-n-butyl [1,2,3] triazolo [4,3-a] chinazolin-5-(4H)-on und dessen Salze ;

1-Piperidinomethyl-4-allyl [1,2,4] triazolo [4,3-a]-chinazolin-5-(4H)-on und dessen Salze.